(19) Europäisches Patentamt
European Patent Office
Office européen des brevets

(11) EP 2 808 397 B1

(12) **EUROPEAN PATENT SPECIFICATION**

(45) Date of publication and mention of the grant of the patent:
**01.11.2017 Bulletin 2017/44**

(51) Int Cl.:
***C12Q 1/68*** *(2006.01)*

(21) Application number: **14170195.3**

(22) Date of filing: **28.05.2014**

# (54) Method for obtaining information on colon cancer and marker and kit for obtaining information on colon cancer

Verfahren zur Gewinnung von Informationen über Darmkrebs und Marker und Kit zur Gewinnung von Informationen über Darmkrebs

Procédé, marqueur et kit permettant d'obtenir des informations sur le cancer du côlon

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2013 JP 2013113425**
**30.04.2014 JP 2014093771**

(43) Date of publication of application:
**03.12.2014 Bulletin 2014/49**

(73) Proprietors:
- **Sysmex Corporation**
  **Kobe-shi, Hyogo 651-0073 (JP)**
- **The University of Tokyo**
  **Bunkyo-ku,**
  **Tokyo 113-8654 (JP)**

(72) Inventors:
- **Sakai, Ayako**
  **Kobe-shi**
  **Hyogo 651-0073 (JP)**
- **Tai, Kaya**
  **Kobe-shi**
  **Hyogo 651-0073 (JP)**
- **Nagae, Genta**
  **Tokyo 113-8654 (JP)**
- **Aburatani, Hiroyuki**
  **Tokyo 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
- **YOUNG-HO KIM ET AL: "Epigenomic Analysis of Aberrantly Methylated Genes in Colorectal Cancer Identifies Genes Commonly Affected by Epigenetic Alterations", ANNALS OF SURGICAL ONCOLOGY, SPRINGER-VERLAG, NE, vol. 18, no. 8, 5 February 2011 (2011-02-05), pages 2338-2347, XP019926925, ISSN: 1534-4681, DOI: 10.1245/S10434-011-1573-Y**
- **MYOUNG SOOK KIM ET AL: "DNA methylation markers in colorectal cancer", CANCER AND METASTASIS REVIEWS, KLUWER ACADEMIC PUBLISHERS, DO, vol. 29, no. 1, 6 February 2010 (2010-02-06), pages 181-206, XP019787671, ISSN: 1573-7233**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 808 397 B1

SEQUENCE LISTING

<110> SYSMEX CORPORATION THE UNIVERSITY OF TOKYO

<120> METHOD FOR OBTAINING INFORMATION ON COLON CANCER AND MARKER AND KIT FOR OBTAINING INFORMATION ON COLON CANCER

<130> SYSM-087-EP

<150> JP2013-113425
<151> 2013-05-29

<150> JP2014-093771
<151> 2014-04-30

<160> 10

<170> PatentIn version 3.5

<210> 1
<211> 4086
<212> DNA
<213> Homo sapiens

<400> 1

```
agatattgaa cacttgttca atatttggat cttttttttt gcaagacaaa attgaatgct      60
agtcacttta taatttgtgc cttctaaaag ggaaaacctt aatcctaagt ttaaaataaa     120
atgtgcactg actacagcat ggtagtagat tattttagta acaagagatt caacaagtct     180
tctcggctag tgctttaaat cgtatatgcc ctattataaa accggtgaaa ccagaaagca     240
aaccgtcctc ctcacactgc ccaaaacaac tgccaataga gcgcatactc atatgtcaaa     300
gccaaaacga tgacagaagt ttggtcttcc tttcagctaa atagtgttaa caggtggtgg     360
ctgggtgaag gcaggagagt gcctgttgct ggccttaggc ctcagataaa taaggaaatg     420
actgaatgaa caatttaaaa ctacacacac tagtcagtgt aactatctag taagttaaat     480
acgccatgac atctaatgat ggcctctgag acagagagca ggctagagcc ggagcgtttg     540
acgtgattac agcagagtgg aggcggttcc ttcctggcta tattagctga aacccaaagc     600
aaatcttgcc ccagcacaag gcaccccttt ctgcgcctgt agtgtgtcag ctatgtgcat     660
tttccttcag gatgagcaaa aatatttcgt catcctcccc gctcctcaac agcgcagaag     720
atgccgaaaa gcaattacag gaccttacag agctgctccg ggggccgcgg gaaacttacc     780
agcatcctgg aaaagacaaa accagtgggg tgcacttggc ctctcctggc tgcagggtgt     840
ggccgccccc acctttcacc ttcccatcct taggaagcaa agtgacccct aagcctagac     900
aaagctctcg aaagcccaaa gcctcgggcc caccggccag ctccccaccc cgctgctggg     960
ccggacaggt gtaggggagg cggacccgcc ccgcagccga ctcacccagc tccagggcct    1020
ggtcgcacct gagcagcgcg gcctccggct gctgctggcg ctgcaggctc cgcgcctggc    1080
ctgccagcct gcgcagccgg cactcggccg ggaagcactt ctccagcagg ccgctcagca    1140
```

```
ccacgttcac gcgccgcacc tgcggccgcg cgggccctgg ctccacgcag cgcttgcaga    1200
ctgtgagccc gcagggcagc gtcaccggct tatgcagcag ccgccggcag cgcgggcagc    1260
cgagcaggtc gcggggcgcg cggggctccg gggccggccc tccctcgccg ggcgcctcgg    1320
gctcgccgcc cgggttctcc gcggacagcg gccggtcgcg caggcccacg gcgcgcacca    1380
ggccgcccgc cagctcttcc agctcctccg gccgcagcgc cccgagccgc gcggcgccgc    1440
ggaacgcgcc cagggcttcg gggaggcggc cggcgcgggc cagcgcgtcc cccagcctca    1500
ggcacaggcc gcggtccggc tgcgccagcc cggctagcat ggagcgaaag agctcggctg    1560
ccatctcgta gtcgcccgcg cggaaggcct cgtcgccctc ctctaagcgc tgggcgatcg    1620
gctccgcgcg gtcgcagcca ggacactggg gcggcggcgg cggcgggacc ggctcggggc    1680
tcatcaccgc ggggctgcga cgacgcgggt ccggagcgaa ggcgcggagc agggaggatg    1740
cgctgctgct gggaactggc cggcgggagc gcggtctcag ccctcgccag cagccacgcg    1800
cgtctggggg cggcgcgctg cgagcggctg agaccgcggg cgggggcggg cgcctggctt    1860
gggcagcgtc ctcagcgcgg tgtgggcggc gagccccgca gggctgcaat cgttccgggg    1920
tgggggccgg gacaggcacc gcgggcgcaa tctgagcccc tgcccacgcg cagcggcctc    1980
tcagtcccgc cggcttaggt aacccaggtc gctgcggtaa cgcagtgacc gcgctccagg    2040
tccgcgtctc ttgcgatgct tcccccactc gcctgagggc tcctgcgcga ctgcgcgcgc    2100
gtcctctgcc tgccgcctcc ccgcagaggt gccggggccc tgggagcagg tggccttggc    2160
cgcgggctgc tggcgcgccg gcaccgcggc acctgctctt ccccagaggc ctggccgccc    2220
ccacaacctg tggctccgct taagcaagaa cccaggaaaa gtcaccaaac gcatcacgca    2280
tctctagctt cgacttagga aattgtccta aatgactggg gaggctgaag tgggcaccca    2340
gaggccccgc ctcagcgagc ttcttctctt aactctaggc tgaggccttt gagaactcat    2400
tttaacagag aacgtggagg gccaagagaa ccagatacca ggaggaggag ggattcgaga    2460
agtggacaat tgtattgtgc ccttgattag gaagcagaaa tagaattaga aatagaattc    2520
taattaatag aattagaata gaaatagaaa gggtgcccta gtatggagta ctgcagacat    2580
aagttgctgg ggagagggag ctaaacgctc cgttggtgta caagcaaggt ttaaattaaa    2640
atcctctgtg cgtattaacc gggttcataa tttctcctat atagcctatg tatttctcct    2700
atgtagcctg ctcccttctt cctgtcgtca cagtatctgt tttatcctgt gtatttgaat    2760
acaataaaat gcattacaat attctagaat tcatactact gttcatattt caagaataaa    2820
ttttttttaaa aaattggaga ggtttttcta catctaaaac tgctggctaa agaaaatata    2880
caatacctcg accacatctg gctttgggaa aggagaaatg cctcattaag tgagagaaca    2940
ttttagacca gttcctaaaa gggaaaaagg ggcaattttt ggccagccct ctagttctac    3000
```

aataaacaaa tgcagagtac ccacctcctt ttccatgaag gatttccccc agctgtctac 3060
ctaacttaat tgataacagt gtactgtttg ggtctctttt tcattactga attatttcaa 3120
attcactttt tcaatcactg tttggatttt tatctgcttt ctttatagaa taacaattca 3180
cttatatttg agggtcattt atccatcaaa ttcacaacca acaatcagga agcaatgggg 3240
aaaaagcttc agagcacaga ccatacagta aagattgtga aaccttatga tctcagatcc 3300
tatttgttct tattttagac aaatatctag caagagaaca gaaagaagct tgccggaggt 3360
atgcctgctg agagccagaa gtgtttaaag caaaaatgtt agtgtgttgt gaggtttata 3420
acacatagaa ataaaatata tcacaaatat aacacaaagg caggtagagg agaaatggca 3480
ggatactgtt gtaaggttct aatgctatat gtgaaatggt ataatgtcac tttaagaaag 3540
actgtaatga gttaaggatg tgtcctctaa ccctaaagca acctcctaaa taacacaaag 3600
agctatagtt taaaagccta tagagatgaa atgaaattgt taaaaaaaac tctattaatc 3660
caaaagaagg cataaaaaga gaggaaggaa acaattagat aaaacaaata gagaacaaat 3720
agcaagatgg caggtttaaa cccaattata tcggttaatg ttcattaaat attgtctaaa 3780
taccataatt aaaaggcaaa gattgtcaaa aggaagaccc agatatatgc tgcctaccaa 3840
aaaagaactt ttaaatatga aaatacaaat aaggtaaagg taaaaggatg gaaaaagatg 3900
ttctagttaa ctctaagcaa aataaatcta gagtgactat attgatagct gacaaagtct 3960
attacatagc caagaacatt gctaggagaa aggacagcca tttcataatg ataaaggtgt 4020
tagttggtca agaggacata atagtcctaa ccgcgtgtgt tcccaataac agagctttca 4080
aatatt 4086

<210> 2
<211> 4133
<212> DNA
<213> Homo sapiens

<400> 2

```
gtgatctgtc cttcaagcac aacatatcag gaggtacacg atgtcagtgc gtcctgttcc      60
tgcaaaatca cacataagtt taaaataatt ttcttcatca aacccttggc ttgttaaaca     120
ttgcttggaa gggtttcaca cgcactctag taggacttta aaaagcagac ccaaagacaa     180
ggaagccaca agctttacag atgctcagga gaaaaatgta gccatttaaa aaattgtaat     240
gtgatagtta tgaaatttcc ctaaattcat tttgcagaat gagagctgta atttaaacct     300
gaagttatcg agagctgaga taaaccacac atcacgtgca atttaatctg gcagggaaac     360
aggccactcg gaattgctaa ctaggcctaa ttcattacat ctcggtgaat gcagtacttt     420
tggtcttacg cgtctttcaa ctcctgcatt aatatattta cccagacctc atggaaaatg     480
tatgggaatg gaaacagaaa tactgtatcc ttgtttaaat cacatttcat tttttaaaca     540
```

```
gctcaaatga ttgctcagaa agatcacacc ttcttccgaa gcatttatga aacaattatt      600
tacattacat taaaaacttt aattgcctca aagcacgtgc tctgagacac aaatattgga      660
aaatcatcaa aaatatccat catgattctc tcttctttat aactcttcta aactttatct      720
tttttataac ccactgtatt gatctattgt cccagaccaa gagtgatgtc agaagtctag      780
gctgggtcat aaaaagaata taatttccat ttgctgtcca taaaagcaaa tcttttgatc      840
agagaaaaag gaaaaacatg tccccttttt ttgagtgggg aagagggtgc ggtgctatgt      900
atcaggggaa taagccagag aggaagggtg acctcacctt taagggtggt ggcagggata      960
ggcaaggggg ttggtgatgg agggggctgg gagtatgagt tccccctgga ggtgacatct     1020
cacctccagg ggaagtggaa acagaggacc tcgagcttgc acagaggcaa aaacaaacaa     1080
acaaacaaac aaacaaacaa acaaaaaagc agacagaaaa atgtaaaaca agaaatccag     1140
tcaccccggt tgtcaagagt ccgaagaatt gcaaaggacc ggaacctccc tctagccatg     1200
ctgaagaccc tctccccaaa gacaatcaca ataaaggaat gtcatgttgc aaagaacgga     1260
gccccggata ataatgatga taacaacagc agaaacctcc gaggttttta aggatccgga     1320
gcccccacag caggggtagg gcaccctcaa cagtcataac aataaaacaa gcccactggg     1380
ggaaaaggcc ctccagaggt ttggttcaga accaaataac aaaacctcca ataatcatca     1440
tcacagtaaa agggtttcac cttgcaaagc tgtgggggag tccctataat tgggttaaag     1500
accccccaga taataacaac agaaatccct attccaaagg acctgaggcc ccgccctcgg     1560
cccccgcccg taaagaaccc tgagctacta ctcagacgaa gccccccctcc ccgccaataa     1620
taataatata atggcgcgga aactgtgggc ggtggtggtg acatttccca cgactcagtg     1680
gcgcccccgg gcggctccca ccctccctcc ggccggcgcg tctacgcagc cccaagtgct     1740
ggctgcgacc ctcggatccc aagcatatct ggcgatggag cggcggcagc ccgaggcacg     1800
tgttgtgtgt gctccgattg caaaagaaaa aaaaatgcat ttcaaactat taactttttt     1860
ttaagcgtgc aatgccggga gctgggggta gacaggtgca agcgggggta ggcgccccgc     1920
gcgcccgcct cccgcagcgc cccctcccaa acttcccttt gcattgatca gcacgttacg     1980
tcagtattga taagtttgca aaaagccaaa gtcaagtgca atcgcgcggc acaccgaccc     2040
cggacttggg ggggagatgc agcgagcgct ccgcgggccc gggagccggc ggcaggcagg     2100
gcagcggcgg cgccggcgcc tctcggagag gctgcctccc cccaacccct cccctcccac     2160
cccatccccg gctgcctccc ccgggcgggc gccgagctcc ccgcccggcg cgctccgccc     2220
gcgccctccg ggggtcgggg gggcgcacgg ctcccgggcc cgttggcggc ggcggcggcg     2280
gcggcgcggc ggcggcgagg ggcagcgggt ggcaggaggc cggagggcgc ccgaggggcc     2340
ccgggccgcg gcgctcaggg cccgggcggc cggcggcggc cccggggctg gggggagtcc     2400
agcccggata ttgagtgcag ccattgagaa aagccaaact cttgtgtgtg cgcgtctcga     2460
```

```
tagcccccaa gatggccgcc aatgtgggat cgatgtttca atattggaag cgatttgatc     2520
tacggcgact ccaggttagt gcgggcagcg ccggccgcgc ggccgtgagg agcccccggg     2580
cgcgccctgg gcgcattggg gaggtccccg ggcgggcagg cggacgccct ggggcggcgg     2640
gcggcggctg ccggggctcg ccggggctcg gccgcccgct gcccatcgat aggtattagg     2700
aattgatctc gccgctgctc tttgttcggt tcagtcatcg attagctgcc gcgaccatgg     2760
agaagcgcta gtgagccctc ggtcggcgga gcggccgagc ggccaggcgg ccgggcgagg     2820
agcggggaag gcaggggcta cggcctcggg caggggcctg ctggcgggaa cccccggcgg     2880
tccccccctga gccgcacttt gcgcgcctcc caacttcgcg gcgcccgggg aggccgcgga    2940
gcgcgccgct tgccagtccg cacccggctg gggctccggc gagggaggga gggagctggc     3000
gggcagggag gaggaggagg agaggaggag gaggaggaga gaggaggagg gagccggggt     3060
tggcgaggag gcggctccgc gccccgccgc tcgccggcac ctcagccttc gccgcccgcc     3120
tggctgcgca gcccggacgc gccgccaccc gggggccgcc gccgccgccg ccagagccgc     3180
cgccgccgga ctggccgcaa gcgccggcag acctcttctc ctcgggccgg ggtcttgggg     3240
ttcgtcttgc ttcttgcctt taccccccccg ccccttccat ccccttccca agtctagatg    3300
cagacgggcg gtggaggtgg gcccgggcgc tcgcaagttt gcgctcctgc ctccagggcg     3360
gtggagagcc gggagcggcg cagagcaggt gactcccagc cctcgggccc aagggaactt     3420
ttaaatgaga gatttccttg cttgttctgc ctcctgcgtt tctccccgct gctcccctcg     3480
tctcctctct cccgtctctg cttttcttct gtttctttcc tctcttccct gctctttctc     3540
tctccttccc tagggcgact cttcgaaacc ccatcacttt ctccccatcc tccctttgtg     3600
ggagtctgtg tgcctcccct agatttggag gtgttctctg cggaccccgt aggagccggg     3660
gctgggaggt ggaatctcag agaacgcacc gtggaggcgc ggctccggcc tctgttcttt     3720
cccggagtgc cccggacgcg cctggcaagg ccccccagcc ttggagtctc gcaaagtctc     3780
ggcgggtgcg gcggggatca ggggcgggaa aatggtgcgg ataacagggc ggttagagca     3840
tcctgccttg accgtgacat tcgcggaaga cgcgagatca tcagtctgga gataaaaagg     3900
gacccacttt tttttttttt tttaatccgc cggcaaatct cgttaagttt cttctgataa     3960
gtggttccag cacccgcgcc ttctccccgt cctggccgct tcccagtccc cgtccttccg     4020
aacgccccac tcctcgctct ccccctcccc caaagccatt gagctgggga gaaaatcggg     4080
gcactgaggc atgcagttaa taactgccga gtgccttgca attccgggtc cgc            4133
```

<210> 3
<211> 21
<212> DNA
<213> Artificial Sequence

<220>
<223> primer

<400> 3
aatttaggtc gttgcggtaa c 21

<210> 4
<211> 19
<212> DNA
<213> Artificial Sequence

<220>
<223> primer

<400> 4
gcaaaaaccc tcaaacgaa 19

<210> 5
<211> 25
<212> DNA
<213> Artificial Sequence

<220>
<223> primer

<400> 5
aaaagttaaa gttaagtgta atcgc 25

<210> 6
<211> 17
<212> DNA
<213> Artificial Sequence

<220>
<223> primer

<400> 6
aacctctccg aaaaacg 17

<210> 7
<211> 33
<212> DNA
<213> Artificial Sequence

<220>
<223> primer

<400> 7
gggatattaa gtggagttat tttggtttta gtt 33

<210> 8
<211> 21
<212> DNA
<213> Artificial Sequence

<220>
<223> primer

<400> 8
ccctcccaac atccttccta a 21

<210> 9
<211> 86
<212> DNA
<213> Artificial Sequence

<220>
<223> bisulfite-treated DNA

<400> 9

**aauuuaggtc gutgcggtaa cguagtgauc gcgutuuagg tucgcgtutu ttgcgatgut 60**

**tuuuuuautc guutgagggu tuutgu 86**

<210> 10
<211> 133
<212> DNA
<213> Artificial Sequence

<220>
<223> bisulfite-treated DNA

<400> 10

**aaaaguuaaa gtuaagtgua atcgcgcggu auaucgauuu cggauttggg ggggagatgu 60**

**agcgagcgut ucgcggguuc gggagucggc gguagguagg guagcggcgg cgucggcguu 120**

**tutcggagag gut 133**

## Description

### TECHNICAL FIELD

The present invention relates to a method for obtaining information on colon cancer in a subject. The present invention also relates to a marker and a kit used in the method.

### BACKGROUND ART

Colon cancer is a collective term for carcinoma located in large intestine (colon, rectum, anus). Colon cancer develops at the surface of intestinal mucosa, invades into the large intestinal wall and finally metastasizes to lymph node or other organs. Screening for colon cancer based on occult blood tests has been widely carried out because early detection of colon cancer allows almost complete treatment of the disease. However, the examination of colon cancer is based on bleeding from the lesion site, and thus there is a possibility that colon cancer which is not accompanied by bleeding may be overlooked. The examination may also give positive results for bleeding due to diseases other than cancer such as hemorrhoid. Thus thorough examination of colon cancer is generally carried out by endoscopy that allows direct observation of the whole intestine. Meanwhile endoscopic examinations put a great burden on subjects because the examinations require dietary restrictions as well as pretreatment using laxatives and an endoscope is inserted into the intestine.

Examination for colon cancer may also be performed by measurement of tumor markers in blood. The tumor markers used for evaluation of metastasis and recurrence and evaluation of treatment efficacy of cancer include CEA (carcinoembryonic antigen), CA19-9 and the like. However, the examination of the tumor markers which have been utilized for examinations of types of cancer different from colon cancer has insufficient sensitivity and specificity to allow the specific screening examination for colon cancer.

Meanwhile, new diagnosis methods for cancer have been recently studied which are based on genetic information. The methods include, for example, ones based on information on methylation of DNAs. The methods use markers which are CpG sites (5'-(CG)-3') in base sequences of certain genes. Based on the analysis of the methylation status of the markers, information such as presence or absence of a cancer cell is obtained, which information may be used as an index for diagnosis of cancer.

Methods for determining cancer utilizing methylation analyses of DNA have been studied and developed for colon cancer. For example, the publication by Hibi K. et al. discloses that genes such as p16 (also referred to as CDKN2A) are highly methylated in tissues from patients with colon cancer (see Non-Patent Literature 1). The publication by Vilkin A. et al. discloses that MLH1 gene is highly methylated in colon cancer tissues with microsatellite instability (see Non-Patent Literature 2). Kim et al. (2011), Annals Surgical Oncol 18(8):2338-47 describe epigenomic analysis of aberrantly methylated genes in colorectal cancer. Kim et al. (2010), Cancer Metastasis Rev 29:181-206 likewise describe DNA methylation markers in colorectal cancer.

Citation List

Non-Patent Literature

Non-Patent Literature 1: Hibi K. et al., Jpn. J. Cancer Res. vol. 93, p.883-887 (2002)
Non-Patent Literature 2: Vilkin A. et al., Cancer. vol. 115, p.760-769 (2009)

### SUMMARY OF INVENTION

Technical Problem

Although some genes indicative of abnormal methylation in colon cancer have been reported as described above, these genes are genes which are also methylated to some extent in a type of cancer different from colon cancer. When methylation analysis is carried out with a marker that is methylated in several types of cancer including colon cancer, information deduced from the analysis result may include not only information on colon cancer but also information on other types of cancer. In this case, even when an analysis result provides information indicating that a sample contains a cancer cell for example, it is difficult to determine whether the cancer cell is derived from colon cancer or from other types of cancer.

For this reason when obtaining information on colon cancer by methylation analysis of a marker, the specificity of the marker to colon cancer is very important. Thus there is a need for a novel marker specific to colon cancer, which allows specific detection of a cancer cell derived from colon cancer.

Accordingly an object of the present invention is to provide a method for obtaining information on a cancer cell derived from colon cancer by identifying such a novel marker and analyzing methylation status of the marker. Another object of the present invention is to provide a kit suitable for use in the method.

Solution to Problem

The present inventors have identified novel markers which are genetic regions specifically methylated in DNAs obtained from cancerous tissues of colon cancer. The present inventors have found that cancer cells derived from colon cancer can be clearly discriminated from other cells (cells of normal tissues, cells of non-cancerous tissues and cancer cells derived from a type of cancer other than colon cancer) based on the result obtained by analyzing the methylation status of the markers, thereby achieving the present invention.

Thus the present invention provides a method for obtaining information on colon cancer comprising the steps of:

preparing a DNA sample from a biological fluid sample collected from a subject;
analyzing methylation status of a CpG site in a promoter region of LONRF2 (Lon peptidase N-terminal domain and ring finger 2) in the DNA sample obtained from the preparation step; and
obtaining information on colon cancer in the subject based on an analysis result obtained from the analyzing step.

The present invention also provides a marker for obtaining information on colon cancer by methylation analysis, which is at least one CpG site selected from CpG sites located in the promoter region of the LONRF2 gene.

The present invention also provides the use of a kit for obtaining information on colon cancer, comprising a primer set for analysis of methylation status of at least one CpG site selected from CpG sites located in the promoter region of the LONRF2 gene.

Also described herein is a marker for obtaining information on colon cancer, which is a polynucleotide obtained by subjecting an isolated DNA to bisulfite treatment, wherein the isolated DNA consists of a base sequence corresponding to the whole or a partial promoter region of the LONRF2 or CUX2 gene and contains at least one CpG site in the promoter region and at least one cytosine not included in CpG sites.

Advantageous Effects of Invention

The present invention can provide a novel marker which allows provision of information on colon cancer in a subject. The present invention can also provide a method and a kit for obtaining information on colon cancer in a subject by analyzing methylation status of the marker.

**BRIEF DESCRIPTION OF DRAWINGS**

FIG. 1(A) is a graph showing the methylation positive rate of the promoter region of the LONRF2 gene calculated from methylation data of cancerous tissues of colon cancer and non-cancerous colonic mucosa tissues;
FIG. 1(B) is a graph showing the methylation positive rate of the promoter region of CUX2 gene calculated from methylation data of cancerous tissues of colon cancer and non-cancerous colonic mucosa tissues;
FIG. 2(A) is a graph showing the methylation positive rate of the promoter region of LONRF2 gene calculated from methylation data of various clinical specimens;
FIG. 2(B) is a graph showing the methylation positive rate of the promoter region of CUX2 gene calculated from methylation data of various clinical specimens;
FIG. 3(A) is a graph showing the methylation positive rate of the promoter region of a known marker gene CDKN2A calculated from methylation data of various clinical specimens;
FIG. 3(B) is a graph showing the methylation positive rate of the promoter region of a known marker gene MLH1 calculated from methylation data of various clinical specimens;
FIG. 4 is an image showing the results of methylation-specific PCR (MSP) amplification of DNA extracted from normal colonic mucosa tissues, and cancerous tissues and non-cancerous colonic mucosa tissues of colon cancer using the respective primer sets for LONRF2 and CUX2;
FIG. 5 is an image showing the results of MSP amplification of DNA extracted from plasma from healthy subjects and plasma from colon cancer patients using the respective primer sets for LONRF2 and CUX2 and a primer set for accuracy control;

FIG. 6(A) is a graph showing the band intensity of amplification products obtained by MSP amplification of DNA extracted from plasma from healthy subjects and plasma from colon cancer patients using a primer set for LONRF2;
FIG. 6(B) is a graph showing the band intensity of amplification products obtained by MSP amplification of DNA extracted from plasma from healthy subjects and plasma from colon cancer patients using a primer set for CUX2;
FIG. 6(C) is a graph showing the band intensity of amplification products obtained by MSP amplification of DNA extracted from plasma from healthy subjects and plasma from colon cancer patients using a primer set for accuracy control;
FIG. 7 is an image showing the results of MSP amplification of DNA extracted from various cancer tissues including colon cancer and normal tissues from various organs using the respective primer sets for LONRF2 and CUX2 and a primer set for accuracy control;
FIG. 8(A) is a graph showing the band intensity of amplification products obtained by MSP amplification of DNA extracted from various cancer tissues including colon cancer and normal tissues from various organs using a primer set for LONRF2;
FIG. 8(B) is a graph showing the band intensity of amplification products obtained by MSP amplification of DNA extracted from various cancer tissues including colon cancer and normal tissues from various organs using a primer set for CUX2;
FIG. 8(C) is a graph showing the band intensity of amplification products obtained by MSP amplification of DNA extracted from various cancer tissues including colon cancer and normal tissues from various organs using a primer set for accuracy control;
FIG. 9 is a schematic view of an example of a determination device for providing information on colon cancer in a subject;
FIG. 10 is a block diagram showing the functionality configuration of the determination device of FIG. 9;
FIG. 11 is a block diagram showing the hardware configuration of the determination device in FIG. 9; and
FIG. 12 is a flow chart of determination for providing information on colon cancer in a subject using the determination device in FIG. 9.

## DESCRIPTION OF EMBODIMENTS

In the method for obtaining information on colon cancer of the present invention (hereinafter also merely referred to as "method"), a DNA sample is first prepared from a biological sample collected from a subject.

In the present embodiments, the biological fluid sample is not particularly limited as far as it is a biological sample containing DNA of a subject and is preferably a sample containing genomic DNA such as a clinical specimen. The clinical specimen may include, for example, body fluid, urine and the like. The body fluid may include blood, serum, plasma, lymph fluid, ascetic fluid, bone marrow aspirate, nipple discharge and the like.

The DNA sample can be prepared by extracting DNA from the biological sample. Methods for extracting DNA from biological samples are well known in the art. Extraction of DNA can be carried out, for example, by mixing the biological sample with a treatment solution containing a surfactant for solubilization of cells or tissues (e.g. sodium cholate, sodium dodecyl sulfate etc.), and subjecting the resulting mixture to a physical procedure (stirring, homogenization, ultrasonication etc.) to release DNA contained in the biological sample into the mixture. In this case, it is preferable to centrifuge the mixture to precipitate cell debris and use the supernatant containing the released DNA in the next step of analyzing. The obtained supernatant may be purified according to well-known methods. DNA can also be extracted and purified from a biological sample by using commercially available kits.

The preparation step preferably further comprises the step of fragmenting the extracted DNA. By fragmenting DNA to have appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. In case of the restriction enzyme treatment, the restriction enzyme may appropriately be selected based on the base sequence of DNA, which may be *Mse*I or *Bam*HI, for example.

According to the present method, the methylation status of a CpG site in a promoter region of LONRF2 in DNA obtained from the preparation step is analyzed.

As used herein, the term "CpG site" means a site of a sequence in which cytosine (C) and guanine (G) are adjacent in this order from 5' to 3'. The letter "p" in "CpG" represents a phosphodiester bond between cytosine and guanine.

As used herein, to "analyze the methylation status" means to analyze the presence or absence of methylation of a CpG site located in a promoter region of LONRF2 or to analyze methylation frequency in the promoter region.

The base sequences *per se* of the promoter regions of the LONRF2 gene is well known in the art. The base sequences can be obtained from well-known databases such as the one provided by the National Center for Biotechnology Information (NCBI) (http://www.ncbi.nlm.nih.gov/). The ID numbers of the above gene is shown in Table 1. The base sequences of the promoter regions of the genes are represented by SEQ ID NO: 1.

Table 1

| **Gene symbol** | Unigene ID | Entrez ID | Transcript ID | **SEQ ID NO:** |
|---|---|---|---|---|
| *LONRF2* | Hs.134244 | 164832 | NM_198461.3 | 1 |

In particular embodiments of the present invention, the analyzing step may be the step of analyzing the presence or absence of methylation of at least one CpG site among CpG sites located in a promoter region of LONRF2. The term "presence or absence of methylation" means whether or not cytosine in a CpG site located in the promoter region is methylated. In a particular embodiment, one CpG site may be analyzed; however, more than one CpG site is preferably analyzed for the presence or absence of methylation. More than one CpG site may be selected from a promoter region of one gene or from each of promoter regions of more than one gene.

In a particular embodiment of the present invention, the analyzing step may be the step of analyzing the methylation frequency in a promoter region of LONRF2. The term "methylation frequency" means a ratio of the number of methylated CpG site(s) relative to the number of CpG site(s) located in the promoter region. In the embodiment, the target for analysis may be the whole promoter region or a part thereof including at least one CpG site. The target for analysis may contain only one CpG site; however it is preferable that the target for analysis contains more than one CpG site. The target for analysis may be selected from any one promoter region among the above genes or from promoter regions of more than one gene.

The positions and numbers of CpG sites located in the promoter region of LONRF2 is already known. Thus the number of methylated CpG site(s) itself in the promoter regions can also be used as the methylation frequency.

The methylation frequency may be a "methylation score" obtained by analyzing DNA with mass spectrometry such as MassARRAY® as described hereinbelow. MassARRAY® allows calculation of the methylation score based on the ratio between the area of a peak derived from a methylated DNA fragment and the area of a peak derived from a non-methylated DNA fragment obtained after measurement of the DNA fragments.

In particular embodiments of the present invention, the target for analysis may be any CpG site(s) (or certain region(s) including the CpG site(s)) in the promoter region of LONRF2 without particular limitation and may be appropriately selected by a person skilled in the art. The positions and numbers of CpG sites located in the promoter regions of the genes are already known. Thus the target CpG site or region may be selected by routine experiments according to the well known analysis methods described hereinbelow.

Various methods are well-known in the art for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, a MeDIP method, non-methylated cytosine converting treatment and the like.

The step of amplifying DNA may include the step of carrying out PCR, quantitative PCR, IVT (*in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In embodiments of the present invention, the analyzing step may be the step of concentrating methylated DNA in DNA obtained in the extracting step by the MeDIP method and analyzing the methylation status of the concentrated methylated DNA. The methylated DNA concentrated by the MeDIP method may be amplified by e.g. IVT amplification and the methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is a substance which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in a base sequence (C and U) by the non-methylated cytosine converting treatment using bisulfite. The non-methylated cytosine converting treatment using bisulfite is referred to as the bisulfite treatment.

When the bisulfite treatment is carried out, the amount (concentration) of bisulfite added is not specifically limited as long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in base sequence from the original sequence. This process is referred to as a bisulfite sequencing method.

The methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in mass (16 Da) due to difference between G and A between the obtained digested fragments is detected on a MALDI-TOF (matrix assisted laser desorption/ionization time-of-flight) mass spectrometer to analyze the methylation status of DNA. This method is referred to as MassARRAY® analysis.

It is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY® can be identified as to the portions of the base sequences in the template DNA from which the peaks originate. For example, when one CpG site is methylated in a DNA fragment, a peak obtained in MassARRAY® shifts to the side with an increased mass for 16 Da. When a DNA fragment containing more than one CpG site is analyzed for example, the DNA fragment having two methylated CpG sites shows a shift of 32 Da and the DNA fragment having three methylated CpG sites shows a shift of 48 Da.

In mass spectrometry such as MassARRAY®, the methylation score of the analyzed DNA fragment can be calculated. For example when a DNA fragment having a certain sequence results in the ratio between the area of the peak of the non-methylated DNA fragment and the area of the peak of the methylated DNA fragment obtained in a resulting chart from the analysis of 1:3, the methylation score of the DNA fragment is 0.75 (= 3/(1+3)). The methylation score is theoretically 1 for a DNA fragment in which all CpG site(s) is methylated and 0 for a DNA fragment without any methylated CpG site.

Methylation status of CpG sites can be analyzed by a methylation-specific PCR (MSP) method. The MSP method is a method in which the methylation status of CpG sites (presence or absence of methylation) is analyzed by amplifying DNA after bisulfite treatment by PCR using a primer set described hereinafter and determining presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of the LONRF2 gene on a substrate. The microarray can be prepared according to well-known methods in the art.

In the analysis using a microarray, DNA extracted from a biological sample is preferably labeled with a labeling substance well-known in the art. Thus, the present method preferably further comprises the step of labeling the extracted DNA. The labeling step is advantageously carried out after the DNA amplifying step because all DNA in the biological sample may be labeled. The labeling substance may include fluorescence substances, haptens such as biotin, radioactive substances and the like. The fluorescence substances may include Cy3, Cy5, FITC, Alexa Fluor™ and the like. Labeling of DNA facilitates measurement of a signal from a probe on the microarray. A method for labeling DNA with the labeling substance is well-known in the art.

The above signal may be any suitable signal depending on the type of microarray. For example, the signal may be an electric signal generated when a DNA fragment hybridizes to a probe on the microarray, or a fluorescence or luminescence signal generated from a labeling substance when DNA to be analyzed is labeled as described above. Detection of a signal can be carried out by using a scanner comprised in a conventional microarray analyzer. The scanner may be, for example, GeneChip® Scanner3000 7G (Affymetrix), Illumina® BeadArray Reader (Illumina) and the like.

In the present method, information on colon cancer in the subject is obtained based on the analysis result obtained in the analyzing step. The information on colon cancer is not particularly limited as long as it may be an index on diagnosis of colon cancer and is preferably information indicative of occurrence or status of colon cancer or both thereof in a subject. The information may include, for example, presence or absence of a cancer cell derived from colon cancer in a biological sample collected from a subject, a possibility of occurrence of colon cancer in a subject, or a risk for future occurrence of colon cancer in a subject. The information on colon cancer in a subject who has already been affected by colon cancer may include prognosis of the subject, a degree of progression (stage) and the like. The information on colon cancer obtained based on the analysis result obtained in the analyzing step does not substantially include information on a type of cancer different from colon cancer because the promoter regions of the above genes which are used as markers in the present method are specifically methylated in cancer cells derived from colon cancer.

In particular embodiments of the present invention, the analyzing step which provides the analysis result indicating presence of a methylated CpG site may provide information indicating the occurrence of colon cancer or indicating that the status of colon cancer is poor (or aggravated).

In another embodiment of the present invention, the above information can be obtained when the methylation frequency obtained in the analyzing step is higher than a certain threshold.

More specifically, the information obtained may be indicative of presence of a cancer cell derived from colon cancer in a biological sample. The information obtained may alternatively indicate that a subject has a high risk for being affected by colon cancer or that a subject has already been affected by colon cancer. For a subject who has already been affected by colon cancer, information obtained may indicate that prognosis of the subject is poor (or aggravated) or that the cancer is in a progressed stage.

When the result from the analyzing step shows the absence of methylated CpG site on the contrary, information suggesting no occurrence of colon cancer or information indicating that colon cancer is in a good (or reduced) status can be obtained. Alternatively the above information can be obtained when the methylation frequency obtained in the analyzing step is lower than a certain threshold. More specifically, the information obtained may be indicative of the absence of a cancer cell derived from colon cancer in a biological sample. The information obtained may alternatively indicate that a subject has a low risk for being affected by colon cancer or that a subject has not been affected by colon cancer. For a subject who has already been affected by colon cancer, information obtained may be indicative of an positive prognosis of the subject or indicate that the cancer is in a relatively early stage.

The threshold is not particularly limited and may be empirically set based on accumulated data on various biological samples. The threshold may alternatively be set as follows. First, methylation frequency is analyzed for DNA extracted respectively from a biological sample which is confirmed to be devoid of cancer cells derived from colon cancer (normal colonic mucosa tissue or normal colonocyte) and a biological sample containing a cancer cell derived from colon cancer. Next, based on the obtained analysis results, a threshold is set within a range that is higher than the methylation frequency of the biological sample devoid of cancer cells and lower than that of the biological sample containing the cancer cell. Preferably, the threshold is set as a value which can highly accurately differentiate between the biological sample devoid of cancer cells and the biological sample containing the cancer cell.

The scope of the present invention also encompasses the use of a marker for obtaining information on colon cancer by methylation analysis (also merely referred to as "marker") in a biological fluid sample collected from a subject. The marker of the present invention is at least one CpG site selected from CpG sites located in the promoter region of the LONRF2 gene.

In particular embodiments of the present invention, the methylation status of the marker in a DNA sample prepared from a biological sample collected from a subject may be analyzed and information on colon cancer in the subject can be obtained based on the analysis result. The analysis of methylation status and obtainment of information on colon cancer are the same as previously described.

The scope of the present invention encompasses the use of a kit for obtaining information on colon cancer (also merely referred to as "kit") in a biological fluid sample collected from a subject. The kit of the present invention comprises a primer set for analysis of the methylation status of at least one CpG site selected from CpG sites located in the promoter regions of the LONRF2 gene.

In particular embodiments of the present invention, the primer set in the kit may be a primer set for the analysis of the methylation status of CpG sites according to mass spectrometry such as MassARRAY® or an analysis method involving PCR amplification such as the MSP method, the bisulfite sequencing method, among which the primer set for mass spectrometry such as MassARRAY® or for the MSP is preferred. The base sequence of the primers in the primer set may be appropriately selected by a person skilled in the art based on the base sequences in the promoter regions. The primer set used for the MSP method may be a primer set of primers respectively having base sequences SEQ ID NOs: 3 and 4.

The scope of the present invention encompasses the use of a polynucleotide obtained by subjecting an isolated DNA to bisulfite treatment, wherein the isolated DNA consists of a base sequence corresponding to the whole or a partial promoter region of LONRF2 and contains at least one CpG site in the promoter region and at least one cytosine not included in CpG sites (also merely referred to as "polynucleotide") as a marker for obtaining information on colon cancer. The term "cytosine not included in CpG sites" may be any cytosine other than those contained in CpG sites and may include, for example, cytosine in a base sequence in which cytosine (C) and adenine (A), thymine (T) or cytosine (C) are adjacent in this order from 5' to 3' (namely CA, CT or CC).

When the isolated DNA is subjected to bisulfite treatment regarding the polynucleotide of the present invention, a non-methylated cytosine in the isolated DNA is converted to uracil while a methylated cytosine is not converted. In particular embodiments of the present invention, information on colon cancer can be obtained by analyzing the methylation status of CpG sites in the polynucleotide. The isolated DNA can be obtained in the same manner as that described for the preparation of the DNA sample. The bisulfite treatment, analysis of methylation status and obtainment of information on colon cancer are also the same as previously described.

The size of the polynucleotide of the present invention is not particularly limited as long as it allows analysis of the methylation status by the MSP method, sequencing or mass spectrometry and is preferably 50 to 200 bases and more preferably 80 to 130 bases. The polynucleotide of the present invention may include a polynucleotide consisting of a base sequence SEQ ID NO: 9 or 10. The polynucleotide consisting of the base sequence SEQ ID NO: 9 or 10 is suitable for analysis of the methylation status by the MSP method.

The present invention also encompasses a system suitable for the provision of information on colon cancer in a subject. The system may be as follows for example.

A system suitable for the provision of information on colon cancer in a subject comprising a computer containing a processor and a memory controlled by the processor, wherein the memory comprises a computer program for enabling the computer to carry out a process comprising the steps of:

obtaining an analysis result on the methylation status of a CpG site located in a promoter region of LONRF2 in a DNA sample derived from the subject; and
providing information on colon cancer in the subject based on the resulting analysis result.

The present invention also encompasses a computer program product for enabling a computer to carry out provision of information on colon cancer in a subject. The computer program product may be as follows for example.

A computer program product for enabling a computer to carry out provision of information on colon cancer in a subject comprising a computer readable medium, wherein the medium comprises a computer program for enabling the computer to carry out a process comprising the steps of:

obtaining an analysis result on the methylation status of a CpG site located in a promoter region of LONRF2 in a DNA sample derived from the subject; and
providing information on colon cancer in the subject based on the resulting analysis result.

The medium may be a non-transitory computer readable medium of the computer program.

An embodiment of a suitable device for carrying out the present method is illustrated by referring to figures. However, the present invention is not limited only to this embodiment. FIG. 9 is a schematic view of an example of a determination device for providing information on colon cancer in a subject. The determination device 1 shown in FIG. 9 comprises a measurement device 2 and a computer system 3 connected to the measurement device 2.

In the present embodiment, the measurement device 2 is a MALDI-TOF mass spectrometer. The measurement device 2 obtains mass spectrometric information such as the time of flight or the mass-to-charge ratio (m/z ratio) of a substance to be analyzed. The measurement device 2 onto which a measurement sample prepared from the DNA sample derived from a subject is mounted obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2 onto which a gel obtained by electrophoresis of a reaction solution after nucleic acid amplification by the MSP method is mounted detects amplification products. The measurement device 2 then obtains the band intensity data of the amplification products and sends the obtained data to the computer system 3.

The computer system 3 comprises a computer main body 3a, an input device 3b and a display 3c for displaying specimen information, determination results and the like. The computer system 3 receives the mass spectrometric information from the measurement device 2. The processor in the computer system 3 executes, based on the mass spectrometric information, a program for providing information on colon cancer in a subject.

FIG. 10 is a block diagram showing the functionality configuration of the determination device of FIG. 9. As shown in FIG. 10, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a threshold necessary for determination and a formula for calculating the methylation score. The calculating unit 303 calculates the methylation score from information obtained at the receiving unit 301 according to the formula stored in the memory unit 302. The determining unit 304 determines whether or not the methylation score calculated at the calculating unit 303 is lower than the threshold stored at the memory unit 302. The output unit 305 outputs the determination result from the determining unit 304 as information on colon cancer in the subject (e.g., presence or absence of a cancer cell derived from colon cancer in the biological sample collected from the subject).

FIG. 11 is a block diagram showing the hardware configuration of the determination device in FIG. 9. As shown in FIG. 11, the computer main body 3a comprises a CPU (Central Processing Unit) 30, ROM (Read Only Memory) 121, ROM 32, a hard disk 33, an input/output interface 34, a read-out device 35, a communication interface 36 and an image output interface 37. The CPU 30, ROM 31, RAM (Random Access Memory) 32, the hard disk 33, the input/output interface 34, the read-out device 35, the communication interface 36 and the image output interface 37 are connected via a bus 38 so as to be able to communicate each other.

The CPU 30 can execute a computer program stored in ROM31 and a computer program loaded with ROM 32. When the CPU 30 executes the application program, the functional blocks described above may be executed. Accordingly the computer system serves as a terminal that is a determination device for providing information on colon cancer in a subject.

ROM 31 is made up with mask ROM, PROM, EPROM, EEPROM or the like. ROM 31 stores the computer program executed by the CPU 30 and data used for the execution.

ROM 32 is made up with SRAM, DRAM or the like. ROM 32 is used for readout of the computer programs stored in ROM 31 and the hard disk 33. ROM 32 is also utilized as a work area when the CPU 30 executes these computer programs.

An operating system to be executed by the CPU 30, computer programs such as application programs (the computer program for providing information on colon cancer in a subject) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analog interface formed by a D/A converter and an A/D converter or the like. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

The process operations carried out by the determination device 1 for providing information on colon cancer in a subject are illustrated hereinafter. FIG. 12 is a flow chart for providing information on colon cancer using the determination device of FIG. 9. An example is herein illustrated in which mass spectrometric information of a nucleic acid in a measurement sample prepared from a DNA sample derived from a subject is used for calculation of a peak area from which a methylation score is calculated and the methylation score is determined as to whether or not it is lower than a threshold. However, the present invention is not limited only to this embodiment.

In the step S1-1, the receiving unit 301 in the determination device 1 receives from the measurement device 2 mass spectrometric information. In the next step S1-2, the calculating unit 303 calculates from the mass spectrometric information received at the receiving unit 301 a peak area which is sent to the memory unit 302. In the step S1-3, the calculating unit 303 calculates the methylation score based on the peak area stored in the memory unit 302 according to the formula stored in the memory unit 302.

In the step S1-4, the determining unit 304 determines whether or not the methylation score calculated at the calculating unit 303 is lower than the threshold stored in the memory unit 302. When the methylation score is lower than the threshold, the determining unit 304 in the step S1-5 sends a determination result indicating that the biological sample collected from the subject does not contain a cancer cell derived from colon cancer to the output unit 305. When the methylation score is not lower than the threshold (i.e., the methylation score is at or higher than the threshold), the determining unit 304 sends in the step S1-6 a determination result indicating that the biological sample collected from the subject contains a cancer cell derived from colon cancer to the output unit 305.

In the step S1-7, the output unit 305 outputs the determination result as information on colon cancer in the subject so that the display 3c displays the result and/or the printer prints out the result. Accordingly, the determination device can provide, to a physician or the like, information assisting the physician or the like to judge whether or not the subject has colon cancer.

The present invention is specifically described hereinafter by way of Examples which do not limit the present invention.

Examples

Reference Example 1: Identification of novel markers utilizing methylation data of cancerous tissues and non-cancerous tissues from colon cancer

(1) Collection of methylation data

In the present Reference Example, methylation data on Infinium HumanMethylation450 BeadChip (Illumina) for cancerous tissues (324 specimens) and non-cancerous colonic mucosa tissues (40 specimens) of colon cancer were collected which are published in TCGA (The Cancer Genome Atlas : http://tcga-data.nci.nih.gov/tcga/tcgaHome2.jsp).

(2) Identification of novel markers

The Infinium HumanMethylation450 BeadChip includes probes that allow quantification of methylation status of 482,421 CpG sites on human genome. The probes are designed to allow quantification of signal intensity of methylated DNA fragments and non-methylated DNA fragments for respective CpG sites by means of hybridization and single base extension reaction. The 265,824 probes corresponding to about 55% of all probes target the regions within 5 kb from the transcription initiation sites of genes (10.1 probes per gene in average). In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in the respective genes was calculated according to the following calculation formula:

$$(\text{mCpG}) = (\text{signal M})/\{(\text{signal M}) + (\text{signal U})\}$$

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for genes in various tissues was calculated according to the following formula:

$$\text{Methylation positive rate (\%)} = (\text{number of methylation positive specimens/total number of specimens}) \times 100$$

For example, for cancerous tissue specimens, the methylation positive rate of a gene can be calculated by "(number of methylation positive specimens among cancerous tissue specimens/total number of cancerous tissue specimens) × 100". The gene which showed a statistically significant difference between cancerous tissue specimens and non-cancerous tissue specimens was identified as a marker which is methylated in cancerous tissues.

As a result of data mining using Infinium HumanMethylation450 BeadChip (Illumina), promoter regions of the LONRF2 and CUX2 genes were identified as markers which are highly methylated in cancerous tissues of colon cancer (see FIGs. 1(A) and 1(B)). These markers may also be referred to as the present markers hereinbelow.

Reference Example 2: Comparison of methylation data between cancer/tumor tissue specimens derived from several types of cancer/tumor, non-cancerous tissue specimens and normal tissue specimens

(1) Collection of methylation data

In the present Reference Example, methylation data of 7 types of cancer/tumor tissue specimens, 7 types of non-cancerous tissue specimens and 19 types of normal tissue specimens were compared. The number of specimens for the respective tissues is shown in the following tables.

Table 2

| Cancer/tumor tissue specimens | |
|---|---|
| Tissue | No. of specimens |
| Brain tumor (brain) | 114 |
| Breast cancer (Breast) | 548 |
| Squamous cell lung cancer (Lung) | 150 |
| Liver cancer (Liver) | 99 |
| Colon cancer (Colon) | 324 |
| Uterine body cancer (Uterus) | 334 |
| Renal clear cell carcinoma (Kidney) | 282 |

Table 3

| Non-cancerous tissue | |
|---|---|
| Tissue | No. of specimens |
| Brain tumor (brain) | 2 |
| Breast cancer (Breast) | 98 |
| Squamous cell lung cancer (Lung) | 43 |
| Liver cancer (Liver) | 19 |
| Colon cancer (Colon) | 40 |
| Uterine body cancer (Uterus) | 36 |
| Renal clear cell carcinoma (Kidney) | 164 |

Table 4

| Tissue | RCAST | Literature 1 | Literature 2 | Total |
|---|---|---|---|---|
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colonic mucosa (Colon) | 2 | 0 | 0 | 2 |
| Normal liver (Liver) | 2 | 0 | 0 | 2 |
| Peripheral blood from healthy subjects (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal gastric mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |
| Normal adrenal gland (Adrenal gland) | 0 | 2 | 0 | 2 |

(continued)

| Tissue | RCAST | Literature 1 | Literature 2 | Total |
|---|---|---|---|---|
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose tissue (Adipose tissue) | 0 | 2 | 0 | 2 |
| Normal heart (Heart) | 0 | 1 | 0 | 1 |
| Various normal blood cell components (WB, PBMC, Gran, CD4+, CD8+, CD14+, CD19+, CD56+, Neu, Eos) | 0 | 0 | 60 | 60 |

In Table 4, the methylation data for the specimens indicated in the column "RCAST" were obtained by the present inventors according to Infinium Methylation Assay using Infinium HumanMethylation450 BeadChip (Illumina). The methylation data for the specimens indicated in the columns "Literature 1" and "Literature 2" were the methylation data published in the literature documents detailed below obtained with Infinium HumanMethylation450 BeadChip (Illumina). The methylation data in this context are the methylation rate (mCpG) of CpG sites in LONRF2 and CUX2 obtained as described in section (2) in Reference Example 1. The average of the positive rate was plotted for the 18 types of normal tissues excluding the normal blood cell components. For normal blood cell components, the average of 60 samples of the respective blood cell components from healthy subjects (6 subjects x 10) was plotted.

- Literature 1: Nazor KL et al., Recurrent variations in DNA methylation in human pluripotent stem cells and their differentiated derivatives. Cell Stem Cell 2012; 10(5): 620-634
- Literature 2: Reinius LE et al., Differential DNA Methylation in Purified Human Blood Cells: Implications for Cell Lineage and Studies on Disease Susceptibility, PLoS One, 7(7) e41361

(2) Comparison of methylation positive rate between cancer/tumor tissue specimens derived from several types of cancer/tumor, non-cancerous tissue specimens and normal tissue specimens.

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the present markers in various tissues was calculated according to the above formula. The results are shown in FIGs. 2(A) and 2(B). In FIG. 2, "Normal tissues" represent, among the tissues indicated in Table 4, the normal tissues excluding 60 specimens of various normal blood cell components and "Normal blood cells" represent the 60 specimens of various normal blood cell components.

FIGs. 2(A) and 2(B) show that all present markers are rarely methylated in other types of cancer or human normal tissues or human normal blood cells and thus are specifically and highly methylated in colon cancer.

Comparative Example 1: Comparison of methylation positive rate between cancer/tumor tissue specimens derived from several types of cancer/tumor, non-cancerous tissue specimens and normal tissue specimens.

The methylation positive rate was calculated for CDKN2A and MLH1 genes (hereinafter referred to as "known markers") which have already been known to be methylated in cancer cells derived from colon cancer in the similar manner as Reference Example 2 in the respective tissues. The results are shown in FIGs. 3(A) and 3(B).

According to FIGs. 3(A) and 3(B), CDKN2A and MLH1 showed low positive rates in colon cancer and methylation thereof was also detected in other types of cancer, and thus CDKN2A and MLH1 have low specificity to colon cancer. Thus known markers have issues in terms of performance as diagnostic markers of colon cancer. Namely it was shown that a search for markers is required to be carried out thereby taking the sensitivity to colon cancer and the specificity in other types of cancer into account.

Example 1: Comparison of methylation data between normal colonic mucosa, non-cancerous colonic mucosa and colon cancer

(1) Biological samples

In the present Example, the biological samples used were cancerous tissues (5 specimens) collected from colon cancer patients. The control samples used were normal colonic mucosa tissues (2 specimens) and non-cancerous colon tissues (2 specimens).

(2) Preparation of measurement samples and control samples

(i) Extraction of genomic DNA

Genomic DNA was extracted from the above tissues with QIAamp DNA Mini Kit (QIAGEN). The genomic DNA contained in the obtained DNA solution was fragmented with Bioruptor (COSMO BIO). The control genomic DNA used was genomic DNA of human peripheral blood lymphocytes. The genomic DNA from human peripheral blood lymphocytes was amplified with GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the non-methylated DNA fragments was subjected to reaction with SssI methylase (New England Biolab) to methylate all cytosines in CG sequences and obtain a solution of methylated DNA fragments (100% methylated DNA).

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) Methylation-specific PCR (MSP)

MSP was carried out on the measurement samples and control samples (DNA after bisulfite treatment) obtained in the above section (2). The composition of PCR reagents, primer sets and reaction conditions for PCR in MSP are shown below.

<PCR reagent>

| | |
|---|---|
| DDW (sterilized water) | 16.75 μL |
| 10 × PCR buffer with $MgCl_2$ (Roche) | 2.5 μL |
| 2 mM dNTP mix | 2.5 μL |
| 10 μM sense primer | 1.0 μL |
| 10 μM anti-sense primer | 1.0 μL |
| Faststart Taq polymerase (Roche) | 0.25 μL |
| Measurement sample or control sample | 1.0 uL |
| Total | 25 μL |

<Primer set>

The primer sets used for MSP are shown in Table 5. These primer sets allow generation of amplification products when DNA in the target regions is methylated (hereinafter also referred to as "primer set for methylation detection"). A primer set for accuracy control was also used which allows judgment on whether or not the bisulfite treatment has been appropriately carried out (see Table 6). The base sequences of bisulfite-converted regions which are amplified with the primer sets for methylation detection for LONRF2 and CUX2 are shown in SEQ ID NOs: 9 and 10, respectively. The base sequences shown in SEQ ID NOs: 9 and 10 respectively represent the regions amplified with the respective primer sets for methylation detection with all CpG sites located therein being methylated.

Table 5

| Gene Name | Primer name | Base sequence | SEQ ID NO: | Size of PCR product (bp) | Annealing temp. (°C) (X) | Cycles (Y) | Amplified gene region |
|---|---|---|---|---|---|---|---|
| LONRF2 | LONRF2_MF | AATTTAGGTCGTTGCGGTAAC | 3 | 86 | 60 | 34 | chr2:100,938,872-100,938,957 |
| | LONRF2_MR | GCAAAAACCCTCAAACGAA | 4 | | | | |
| CUX2 | CUX2_MF | AAAAGTTAAAGTTAAGTGTAATCGC | 5 | 133 | 64 | 34 | chr12:111,471,511-111,471,643 |
| | CUX2_MR | AACCTCTCCGAAAAACG | 6 | | | | |

Table 6

| Base sequence of primers | | SEQ ID NO: | Size of PCR product (bp) | Annealing temp. (°C) | Cycles |
|---|---|---|---|---|---|
| **Forward** | GGGATATTAAGTGGAGTTATTTTGGTTTTAGTT | 7 | 129 | 60 | 40 |
| **Reverse** | CCCTCCCAACATCCTTCCTAA | 8 | | | |

<PCR reaction conditions>

95°C for 6 minutes;
Y cycles of 95°C for 30 seconds, X°C for 30 seconds and 72°C for 30 seconds;
72°C for 7 minutes; and
keep at 16°C.

In the above reaction conditions, "X" and "Y" respectively represent the annealing temperature and the number of cycles as indicated in Tables 5 and 6.

(4) Analysis of results of methylation-specific PCR (MSP)

The amplification product from MSP was verified by 2% agarose gel electrophoresis. The results are shown in FIG. 4. In this figure, "0" and "100" under "control" represent the 0% methylation control sample and the 100% methylation control sample, respectively.

In PCR using the primer set for accuracy control, bands were detected for all samples as shown in FIG. 4. This shows that bisulfite treatment of the samples was appropriately carried out. In PCR using the primer sets for methylation detection, bands derived from methylated CpGs were not detected for any normal colonic mucosae or non-cancerous tissues. In contrast, PCR of colon cancer tissue samples resulted in detection of bands in 5 samples among 5 samples for both markers of LONRF2 and CUX2. Accordingly it is indicated that in methylation analysis of the present markers by MSP method, methylation of the present markers and colon cancer are correlated as the result of the Infinium method from Reference Example 1. Namely it is suggested that LONRF2 and CUX2 are markers with high specificity such that they are highly methylated in colon cancer but methylation thereof is not detected in normal tissues and non-cancerous tissues.

Example 2: Comparison of methylation data between plasma from healthy subjects and plasma from colon cancer patients

(1) Biological samples

The biological samples used in the present Example were peripheral blood (6 specimens) obtained from colon cancer patients. The control samples used were peripheral blood (3 specimens) collected from healthy subjects.

(2) Preparation of measurement samples and control samples

(i) Extraction of genomic DNA

Plasma was obtained from peripheral blood (2 ml) according to the conventional method. Genomic DNA was extracted from the plasma with QIAamp Circulating Nucleic Acid Kit (QIAGEN). The genomic DNA for control used was genomic DNA of human peripheral blood lymphocytes. In the same manner as in Example 1, a solution of the non-methylated DNA fragments (0% methylated DNA) and a solution of methylated DNA fragments (100% methylated DNA) were obtained from the genomic DNA of human peripheral blood lymphocytes.

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) MSP

MSP was carried out on the measurement samples and control samples (DNA after bisulfite treatment) obtained in the above section (2). The primer sets used for MSP in the present Example are the same as Example 1. The composition of the PCR reagent and reaction conditions for PCR are shown below.

<PCR reagent>

| | |
|---|---|
| DW (sterilized water) | 10.88 μL |
| 10 × PCR buffer with $MgCl_2$ (Roche) | 1.5 μL |
| 10 mM dNTP mix | 0.3 μL |
| 10 μM sense primer | 0.6 μL |
| 10 μM anti-sense primer | 0.6 μL |
| Faststart Taq polymerase (Roche) | 0.12 μL |
| Measurement sample or control sample | 1.0 μL |
| Total | 15 μL |

<PCR reaction conditions>

95°C for 6 minutes;
Y cycles of 95°C for 30 seconds, 60°C for 30 seconds and 72°C for 30 seconds;
72°C for 7 minutes; and
keep at 16°C.

In the above reaction conditions, "Y" is 50 (cycles) for the primer sets for CUX2 and LONRF2 and 45 (cycles) for the primer set for accuracy control.

(4) Analysis of results of MSP

The amplification product from MSP was verified by 2% agarose gel electrophoresis. The results are shown in FIG. 5. In this figure, "0" and "100" under "control" represent the 0% methylation control sample and the 100% methylation control sample, respectively. The band intensity of the amplification products in FIG. 5 was also measured. The results are shown in FIGs. 6(A) to 6(C).

In PCR using the primer set for accuracy control, bands were detected for all samples. This reveals that bisulfite treatment of the samples was appropriately carried out. In PCR using the primer sets for methylation detection, bands derived from methylated CpGs were not detected for plasma samples from healthy subjects. In contrast, PCR of plasma samples from colon cancer patients resulted in detection of bands in 4 samples among 6 samples for LONRF2 and 3 samples among 6 samples for CUX2. Accordingly it is indicated that the present markers are highly methylated in patients with colon cancer and are not detected to be methylated in healthy subjects even when the biological samples used were peripheral blood. This suggests that the present markers have high specificity and are promising as blood CTG diagnostic markers.

Example 3: Comparison of methylation data between various cancer tissues including colon cancer and normal tissues

(1) Biological samples

The biological samples used in the present Example were tissue samples collected from patients with various types of cancer including colon cancer and various normal organ tissues. The details of the samples are shown in Table 7. In Table 7, "T" represents a tumor tissue, "N" represents a normal tissue and "PBLN" represents peribronchial lymph node.

Table 7

| Original tissue | | No. of specimens | Source | Symbol |
|---|---|---|---|---|
| Colon | Cancer | 2 | Purchased from BioChain Institute, Inc. | T1 |
| | | | | T2 |
| | Normal | 3 | | N1 |
| | | | | N2 |
| | | | | N3 |
| Brain | Cancer | 1 | The University of Tokyo | T |
| | Normal | 2 | Purchased from BioChain Institute, Inc. | N1 |
| | | | | N2 |
| Lung | Cancer | 1 | Purchased from BioChain Institute, Inc. | T |
| | Normal | 2 | The University of Tokyo | N1 |
| | | | | N2 |
| Mammary gland | Cancer | 1 | Purchased from BioChain Institute, Inc. | T |
| | Normal | 2 | | N1 |
| | | | | N2 |
| Liver | Cancer | 1 | Purchased from BioChain Institute, Inc. | T |
| | Normal | 2 | The University of Tokyo | N1 |
| | | | | N2 |
| Kidney | Cancer | 1 | Purchased from BioChain Institute, Inc. | T |
| | Normal | 2 | The University of Tokyo | N1 |
| | | | | N2 |
| Corpus uteri | Cancer | 1 | The University of Tokyo | T |
| | Normal | 2 | | N1 |
| | | | | N2 |
| Bladder | Cancer | 1 | Purchased from BioChain Institute, Inc. | T |
| | Normal | 1 | | N |
| Cervix uteri | Normal | 1 | Purchased from BioChain Institute, Inc. | Cervix |
| Testis | Normal | 1 | Purchased from BioChain Institute, Inc. | Testis |
| Blood cell | Normal | 1 | Purchased from BioChain Institute, Inc. | PBLN |
| Stomach | Cancer | 1 | Purchased from BioChain Institute, Inc. | T |
| | Normal | 1 | | N |

(2) Preparation of measurement samples and control samples

(i) Extraction of genomic DNA

Genomic DNA was extracted from the respective tissue samples in the similar manner as Example 1. The genomic DNA for control used was genomic DNA of human peripheral blood lymphocytes. In the same manner as in Example 1, a solution of the non-methylated DNA fragments (0% methylated DNA) and a solution of methylated DNA fragments (100% methylated DNA) were obtained from the genomic DNA of human peripheral blood lymphocytes.

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) MSP

MSP was carried out on the measurement samples and control samples (DNA after bisulfite treatment) obtained in the above section (2). The primer sets used for MSP in the persent Example are the same as those used in Example 1. The composition of the PCR reagent and PCR reaction conditions are shown below:

<PCR reagent>

| | |
|---|---|
| DW (Sterilized water) | 18.8 μL |
| 10 × PCR buffer with $MgCl_2$ (Roche) | 2.5 μL |
| 10 mM dNTP mix | 0.5 μL |
| 10 μM sense primer | 1.0 μL |
| 10 μM anti-sense primer | 1.0 μL |
| Faststart Taq polymerase (Roche) | 0.2 μL |
| Measurement sample or control sample | 1.0 μL |
| Total | 25 μL |

<PCR reaction conditions>

95°C for 6 minutes;
36 cycles of 95°C for 30 seconds, 62°C for 15 seconds and 72°C for 30 seconds;
72°C for 7 minutes; and
keep at 16°C.

(4) Analysis of results of MSP

The amplification product from MSP was verified by 2% agarose gel electrophoresis. The results are shown in FIG. 7. "NC" and "PC" in the figure respectively represent the 0% methylation control sample and the 100% methylation control sample. "rCpG" represents the amplification product using the primers for accuracy control. The band intensity of the amplification products in FIG. 7 was also measured. The results are shown in FIGs. 8(A) to 8(C).

FIG. 7 shows that in PCR using the primer set for accuracy control, bands were detected for all samples and thus the bisulfite treatment of the samples was appropriately carried out. In PCR using the primer sets for methylation detection, bands derived from methylated CpGs were not detected for samples of cancer tissues other than colon cancer and normal tissues. In contrast, PCR for the samples of colon cancer tissues resulted in detection of bands in 1 specimen among 2 specimens for both markers of LONRF2 and CUX2. FIGs. 8(A) to 8(C) show that from comparison of the band intensity for the samples of cancer tissues other than colon cancer and normal tissues with the band intensity from PCR using the primer set for accuracy control, the band intensity from PCR using the primer sets for methylation detection corresponded to the background. From these results, it is suggested that the present markers are specific for colon cancer.

## Claims

1. A method for obtaining information on colon cancer in a subject comprising the steps of:

    preparing a DNA sample from a biological fluid sample collected from the subject;
    analyzing the methylation status of a CpG site in a promoter region of the LONRF2 gene in the DNA sample obtained from the preparation step; and
    obtaining information on colon cancer in the subject based on an analysis result obtained from the analyzing step.

2. The method according to claim 1, wherein the analyzing step is the step of analyzing the presence or absence of methylation of at least one CpG site.

3. The method according to claim 2, wherein
the information on colon cancer in the subject is the presence or absence of a cancer cell derived from colon cancer in the biological sample collected from the subject, and
the step of obtaining information is the step of obtaining information indicating that the biological sample contains a cancer cell derived from colon cancer when the analysis result shows that there is a methylated CpG site.

4. The method according to claim 1, wherein the analyzing step is the step of analyzing methylation frequency.

5. The method according to claim 4, wherein
the information on colon cancer in the subject is the presence or absence of a cancer cell derived from colon cancer in the biological sample collected from the subject, and

the step of obtaining information is the step of obtaining information indicating that the biological sample contains a cancer cell derived from colon cancer when the methylation frequency obtained from the analyzing step is higher than a predetermined threshold.

**6.** The method according to claim 1, wherein the analysis result is obtained by using a marker for obtaining information on colon cancer by methylation analysis, which is at least one CpG site selected from CpG sites located in promoter region of the LONRF2 gene.

**7.** The method according to claim 1, wherein the analysis result is obtained by using a kit for obtaining information on colon cancer, comprising a primer set for analysis of methylation of at least one CpG site selected from CpG sites located in promoter region of the LONRF2 gene.

**8.** The method according to claim 7, wherein the primer set is a primer set for analyzing methylation status of the CpG site by at least one method selected from mass spectrometry and methylation-specific PCR method.

**9.** The method according to claim 8, wherein the primer set is at least one selected from a primer set of primers respectively having base sequences SEQ ID NOs: 3 and 4.

**10.** Use of a kit for obtaining information on colon cancer in a biological fluid sample collected from a subject, said kit comprising a primer set for analysis of methylation of at least one CpG site selected from CpG sites located in promoter region of the LONRF2 gene.

**11.** Use of the kit according to claim 10, wherein the primer set is a primer set for analyzing methylation status of the CpG site by at least one method selected from mass spectrometry and methylation-specific PCR method.

**12.** Use of the kit according to claim 11, wherein the primer set is at least one selected from a set of primers respectively having base sequences SEQ ID NOs: 3 and 4.

**13.** Use of a marker for obtaining information on colon cancer in a biological fluid sample collected from a subject, which marker is a polynucleotide obtained by subjecting an isolated DNA to bisulfite treatment, wherein the isolated DNA consists of a base sequence corresponding to the whole or a partial promoter region of the LONRF2 gene and contains at least one CpG site in the promoter region and at least one cytosine not included in CpG sites.

**14.** Use of the marker according to claim 13, wherein the marker is a polynucleotide consisting of SEQ ID NO: 9.

## Patentansprüche

**1.** Verfahren zum Erhalten von Informationen über Kolonkrebs in einem Patienten, das die folgenden Schritte umfasst:

Herstellen einer DNA-Probe aus einer von dem Patienten gesammelten Probe von biologischer Flüssigkeit,
Analysieren des Methylierungsstatus einer CpG-Stelle in einer Promotorregion des LONRF2-Gens in der aus dem Herstellungsschritt erhaltenen DNA-Probe und
Erhalten von Informationen über Kolonkrebs in dem Patienten auf der Basis eines aus dem Analyseschritt erhaltenen Analyseergebnisses.

**2.** Verfahren nach Anspruch 1, wobei es sich bei dem Analyseschritt um den Schritt der Analyse der Anwesenheit oder Abwesenheit einer Methylierung von mindestens einer CpG-Stelle handelt.

**3.** Verfahren nach Anspruch 2, wobei
es sich bei den Informationen über Kolonkrebs in dem Patienten um die Anwesenheit oder Abwesenheit einer von Kolonkrebs herrührenden Krebszelle in der von dem Patienten gesammelten biologischen Probe handelt und
der Schritt des Erhaltens von Informationen der Schritt des Erhaltens von Informationen ist, der darauf hindeutet, dass die biologische Probe eine von Kolonkrebs herrührende Krebszelle enthält, wenn das Analyseergebnis zeigt, dass es eine methylierte CpG-Stelle gibt.

**4.** Verfahren nach Anspruch 1, wobei der Analyseschritt der Schritt der Analyse der Methylierungshäufigkeit ist.

5. Verfahren nach Anspruch 4, wobei
es sich bei den Informationen über Kolonkrebs in dem Patienten um die Anwesenheit oder Abwesenheit einer von Kolonkrebs herrührenden Krebszelle in der von dem Patienten gesammelten biologischen Probe handelt und
der Schritt des Erhaltens von Informationen der Schritt des Erhaltens von Informationen ist, der darauf hindeutet, dass die biologische Probe eine von Kolonkrebs herrührende Krebszelle enthält, wenn die aus dem Analyseschritt erhaltene Methylierungshäufigkeit höher als ein zuvor festgelegter Schwellwert ist.

6. Verfahren nach Anspruch 1, wobei das Analyseergebnis durch Verwendung eines Markers zum Erhalten von Informationen über Kolonkrebs mittels Methylierungsanalyse erhalten wird, bei dem es sich um mindestens eine CpG-Stelle, ausgewählt aus in der Promotorregion des LONRF2-Gens lokalisierten CpG-Stellen, handelt.

7. Verfahren nach Anspruch 1, wobei das Analyseergebnis durch Verwendung eines Kits zum Erhalten von Informationen über Kolonkrebs erhalten wird, das einen Primersatz für die Analyse von Methylierung mindestens einer CpG-Stelle, ausgewählt aus in der Promotorregion des LONRF2-Gens lokalisierten CpG-Stellen, umfasst.

8. Verfahren nach Anspruch 7, wobei es sich bei dem Primersatz um einen Primersatz für die Analyse des Methylierungsstatus der CpG-Stelle durch mindestens ein Verfahren, ausgewählt aus Massenspektrometrie und einem methylierungsspezifischem PCR-Verfahren, handelt.

9. Verfahren nach Anspruch 8, wobei der Primersatz mindestens einer ist, der aus einem Primersatz von Primern, die die Basensequenzen SEQ ID NO: 3 bzw. 4 besitzen, ausgewählt ist.

10. Verwendung eines Kits zum Erhalten von Informationen über Kolonkrebs in einer von einem Patienten gesammelten Probe von biologischer Flüssigkeit, wobei das Kit einen Primersatz für die Analyse von Methylierung mindestens einer CpG-Stelle, ausgewählt aus in der Promotorregion des LONRF2-Gens lokalisierten CpG-Stellen, umfasst.

11. Verwendung des Kits nach Anspruch 10, wobei es sich bei dem Primersatz um einen Primersatz für die Analyse des Methylierungsstatus der CpG-Stelle durch mindestens ein Verfahren, ausgewählt aus Massenspektrometrie und einem methylierungsspezifischem PCR-Verfahren, handelt.

12. Verwendung des Kits nach Anspruch 11, wobei der Primersatz mindestens einer ist, der aus einem Satz von Primern, die die Basensequenzen SEQ ID NO: 3 bzw. 4 besitzen, ausgewählt ist.

13. Verwendung eines Markers zum Erhalten von Informationen über Kolonkrebs in einer von einem Patienten gesammelten Probe von biologischer Flüssigkeit, wobei der Marker ein Polynukleotid ist, das erhalten wird, indem eine isolierte DNA einer Bisulfit-Behandlung unterzogen wird, wobei die isolierte DNA aus einer Basensequenz besteht, die der gesamten oder einer partiellen Promotorregion des LONRF2-Gens entspricht und mindestens eine CpG-Stelle in der Promotorregion und mindestens ein Cytosin, das nicht in CpG-Stellen enthalten ist, enthält.

14. Verwendung des Markers nach Anspruch 13, wobei der Marker ein Polynukleotid ist, das aus SEQ ID NO: 9 besteht.

## Revendications

1. Procédé d'obtention d'informations sur un cancer du côlon chez un sujet comprenant les étapes de :

   préparation d'un échantillon d'ADN à partir d'un échantillon de liquide organique prélevé chez le sujet ;
   analyse de l'état de méthylation d'un site CpG dans une région promoteur du gène LONRF2 dans l'échantillon d'ADN obtenu à l'issue de l'étape de préparation ; et
   obtention d'informations sur le cancer du côlon chez le sujet sur la base du résultat de l'analyse obtenu à l'issue de l'étape d'analyse.

2. Procédé selon la revendication 1, dans lequel l'étape d'analyse est une étape visant à s'assurer de la présence ou de l'absence d'au moins un site CpG méthylé.

3. Procédé selon la revendication 2, dans lequel
les informations sur le cancer du côlon chez le sujet correspondent à la présence ou à l'absence d'une cellule cancéreuse issue d'un cancer du côlon dans l'échantillon biologique prélevé chez le sujet,

et
l'étape d'obtention d'informations est une étape d'obtention d'informations indiquant que l'échantillon biologique contient une cellule cancéreuse issue d'un cancer du côlon lorsque le résultat de l'analyse montre la présence d'un site CpG méthylé.

4. Procédé selon la revendication 1, dans lequel l'étape d'analyse est une étape d'analyse de la fréquence de méthylation.

5. Procédé selon la revendication 4, dans lequel
les informations sur le cancer du côlon chez le sujet correspondent à la présence ou à l'absence d'une cellule cancéreuse issue d'un cancer du côlon dans l'échantillon biologique prélevé chez le sujet,
et
l'étape d'obtention d'informations consiste en une étape d'obtention d'informations indiquant que l'échantillon biologique contient une cellule cancéreuse issue d'un cancer du côlon lorsque la fréquence de méthylation obtenue à l'issue de l'étape d'analyse est supérieure à un seuil prédéterminé.

6. Procédé selon la revendication 1, dans lequel le résultat de l'analyse est obtenu à l'aide d'un marqueur pour l'obtention d'informations sur le cancer du côlon par analyse de la méthylation, qui consiste en au moins un site CpG choisi parmi les sites CpG situés dans la région promoteur du gène LONRF2.

7. Procédé selon la revendication 1, dans lequel le résultat de l'analyse est obtenu à l'aide d'un kit pour obtention d'informations sur le cancer du côlon, comprenant un ensemble d'amorces pour l'analyse de la méthylation d'au moins un site CpG choisi parmi les sites CpG situés dans la région promoteur du gène LONRF2.

8. Procédé selon la revendication 7, dans lequel l'ensemble d'amorces est un ensemble d'amorces pour analyse de l'état de méthylation du site CpG par au moins un procédé choisi parmi la spectrométrie de masse et le procédé de PCR spécifique de l'étude de la méthylation.

9. Procédé selon la revendication 8, dans lequel l'ensemble d'amorces est au moins un ensemble d'amorces choisi parmi un ensemble d'amorces présentant respectivement les séquences de bases SEQ ID NO : 3 et 4.

10. Utilisation d'un kit pour l'obtention d'informations sur le cancer du côlon dans un échantillon de liquide organique prélevé chez un sujet, ledit kit comprenant un ensemble d'amorces pour l'analyse de la méthylation d'au moins un site CpG choisi parmi les sites CpG situés dans la région promoteur du gène LONRF2.

11. Utilisation du kit selon la revendication 10, dans laquelle l'ensemble d'amorces est un ensemble d'amorces pour analyse de l'état de méthylation du site CpG par au moins un procédé choisi parmi la spectrométrie de masse et le procédé de PCR spécifique de l'étude de la méthylation.

12. Utilisation du kit selon la revendication 11, dans laquelle l'ensemble d'amorces est au moins un ensemble d'amorces choisi parmi un ensemble d'amorces présentant respectivement les séquences de bases SEQ ID NO : 3 et 4.

13. Utilisation d'un marqueur pour l'obtention d'informations sur le cancer du côlon dans un échantillon de liquide organique prélevé chez un sujet, lequel marqueur est un polynucléotide obtenu en soumettant un ADN isolé à un traitement par le bisulfite, dans laquelle l'ADN isolé est constitué d'une séquence de bases correspondant à l'intégralité ou à une partie de la région promoteur du gène LONRF2 et contient au moins un site CpG dans la région promoteur et au moins une cytosine non incluse dans les sites CpG.

14. Utilisation du marqueur selon la revendication 13, dans laquelle le marqueur est un polynucléotide constitué de SEQ ID NO : 9.

FIG. 1(A)

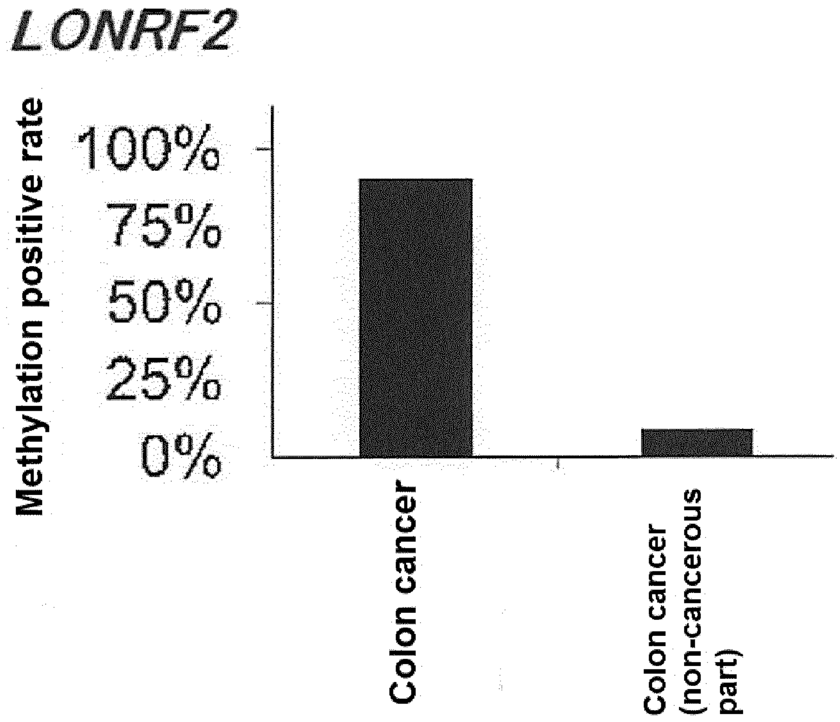
LONRF2
Methylation positive rate
100%
75%
50%
25%
0%
Colon cancer
Colon cancer (non-cancerous part)

FIG. 1(B)

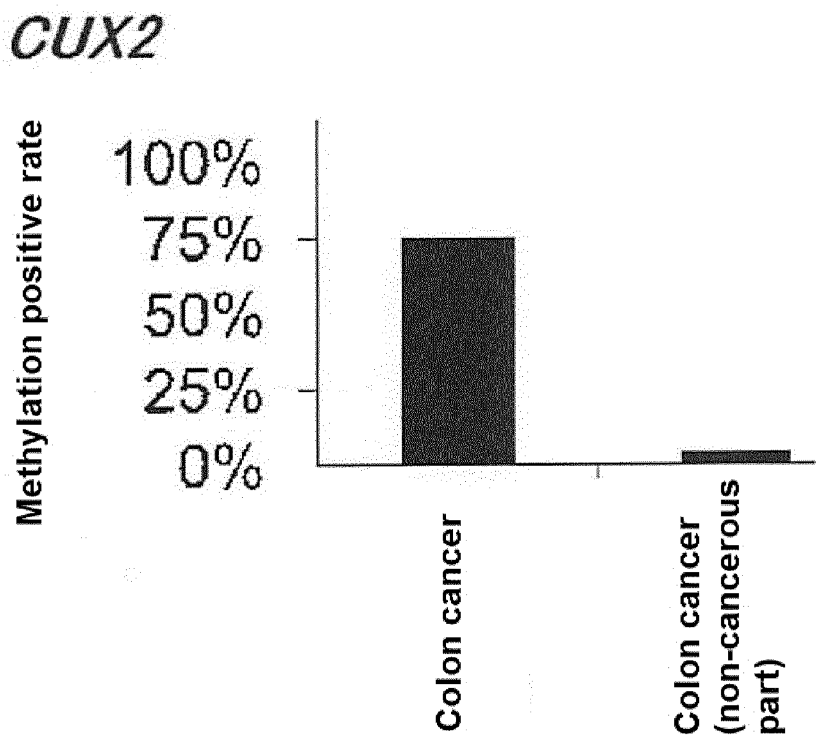
CUX2
Methylation positive rate
100%
75%
50%
25%
0%
Colon cancer
Colon cancer (non-cancerous part)

FIG. 2(A)

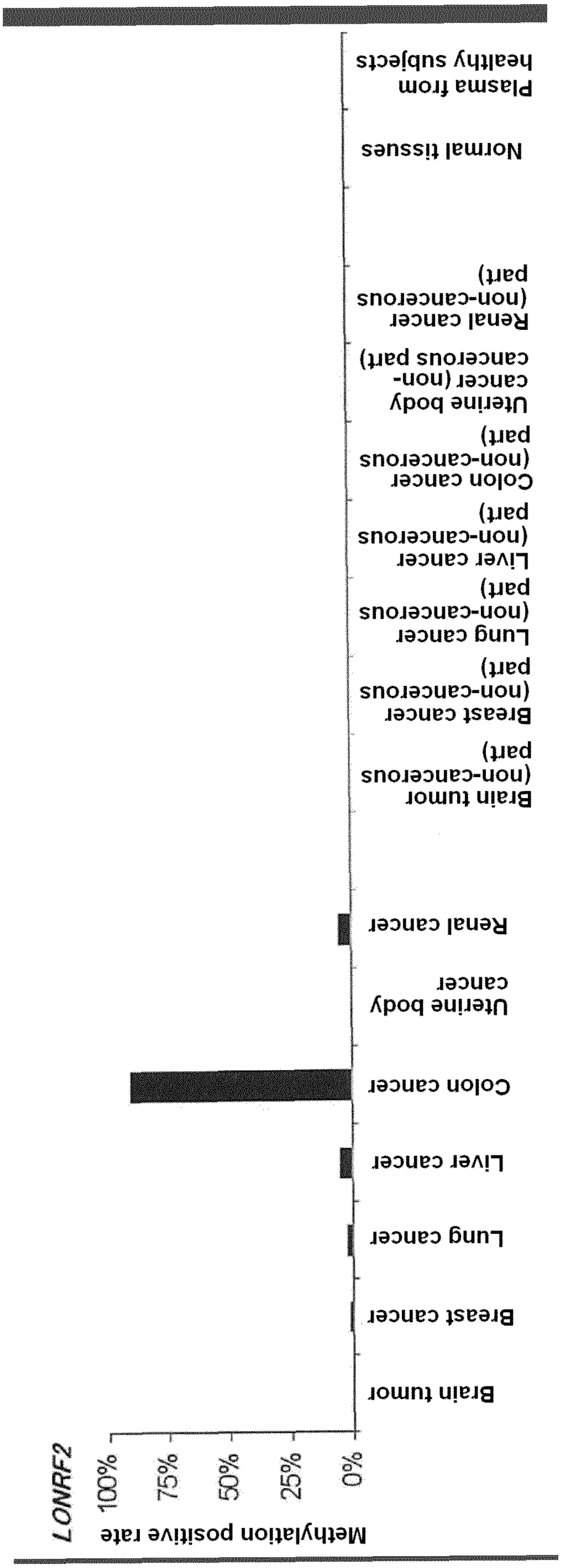
LONRF2
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Plasma from healthy subjects

FIG. 2(B)

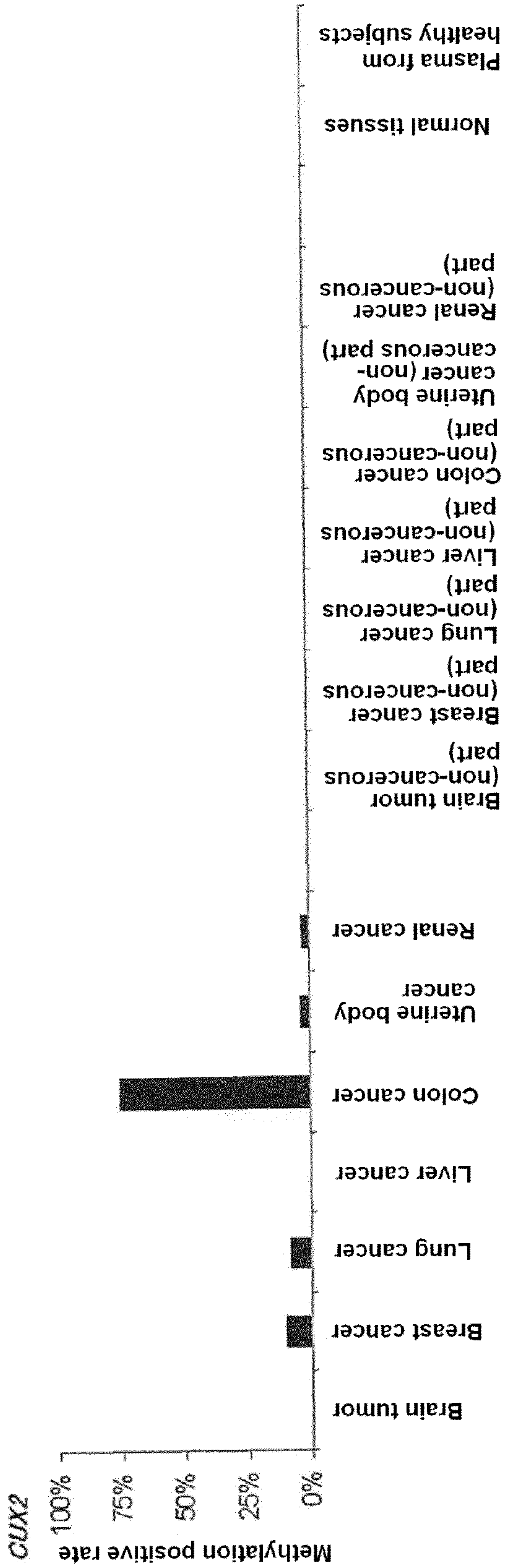
CUX2
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Plasma from healthy subjects

FIG. 3(A)

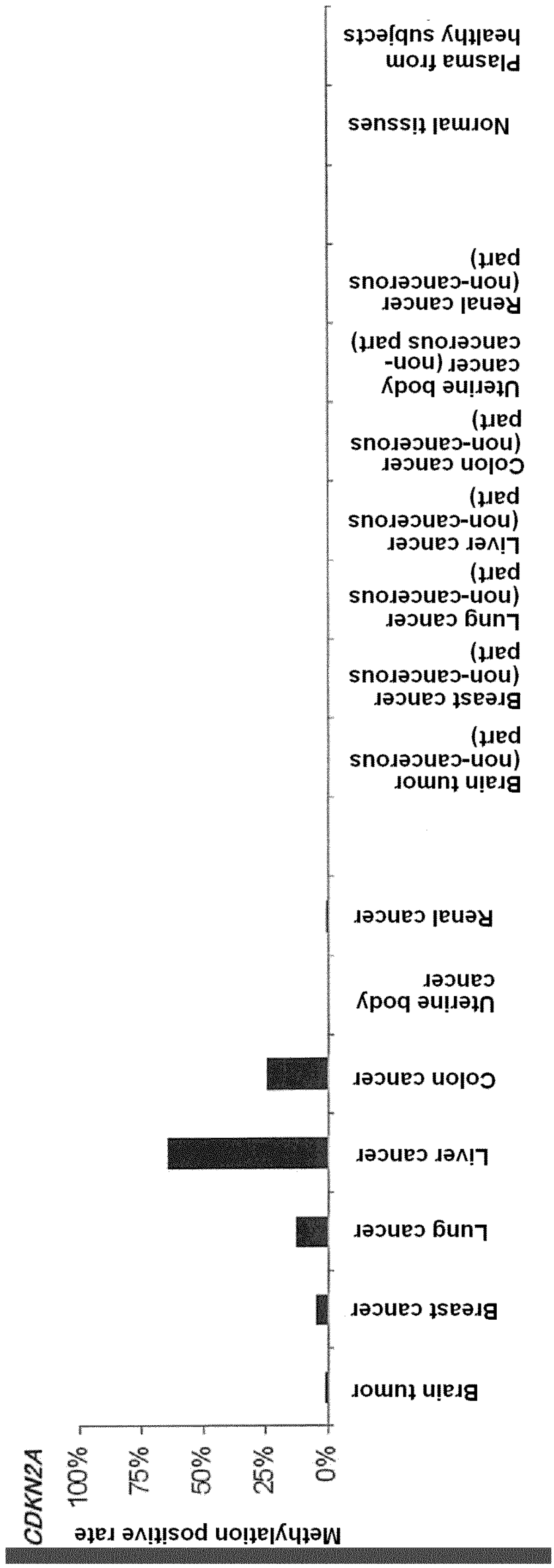
CDKN2A
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Plasma from healthy subjects

FIG. 3(B)

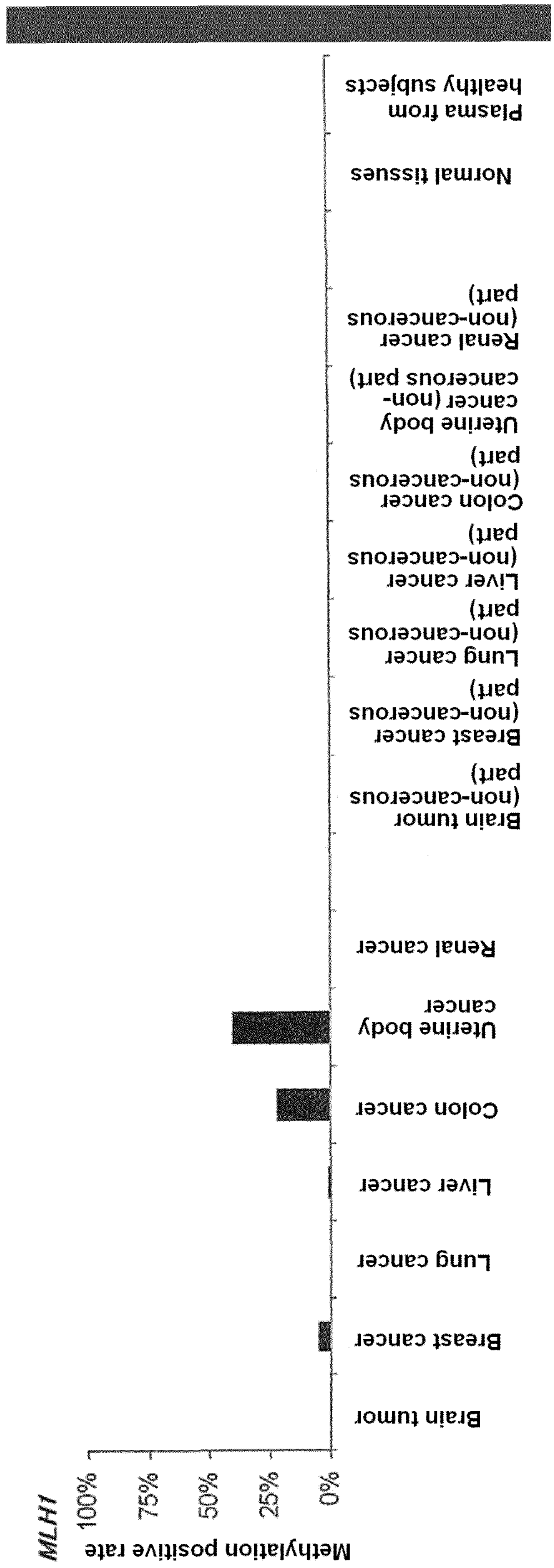
MLH1
Methylation positive rate
100%
75%
50%
25%
0%
Brain tumor
Breast cancer
Lung cancer
Liver cancer
Colon cancer
Uterine body cancer
Renal cancer
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Plasma from healthy subjects

FIG. 4

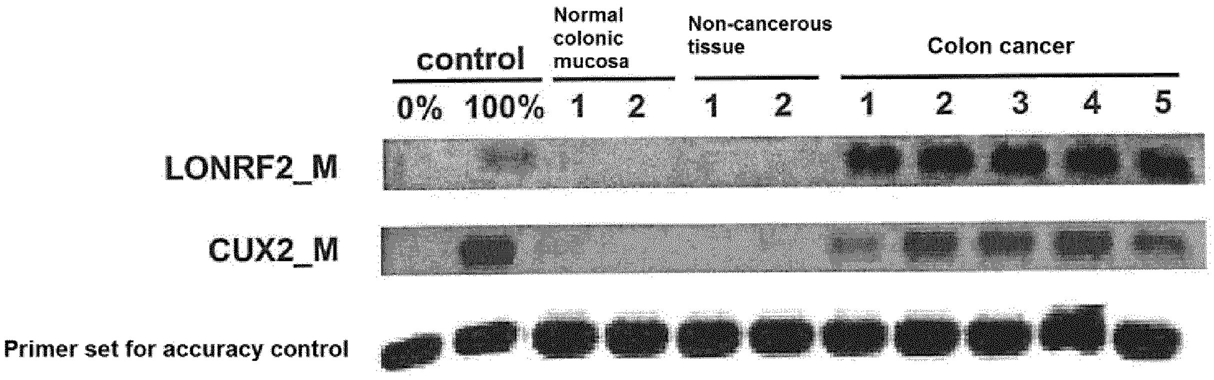


FIG. 5

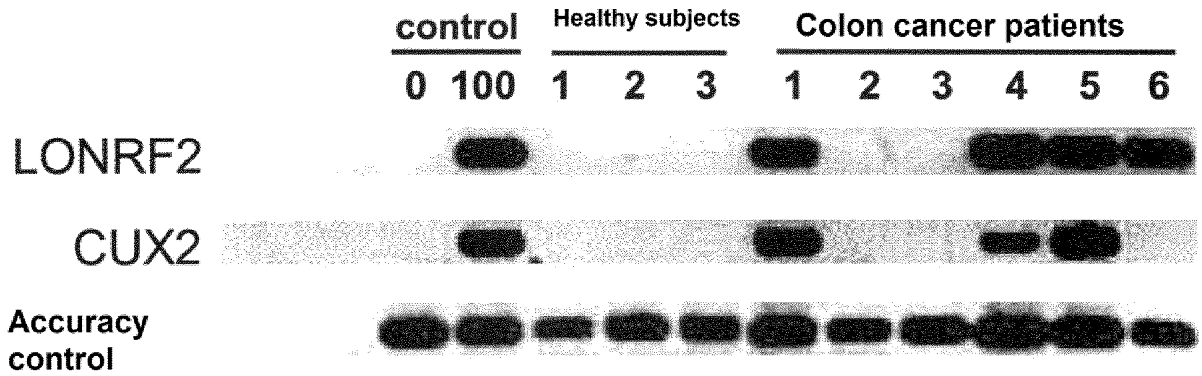


FIG. 6(A)

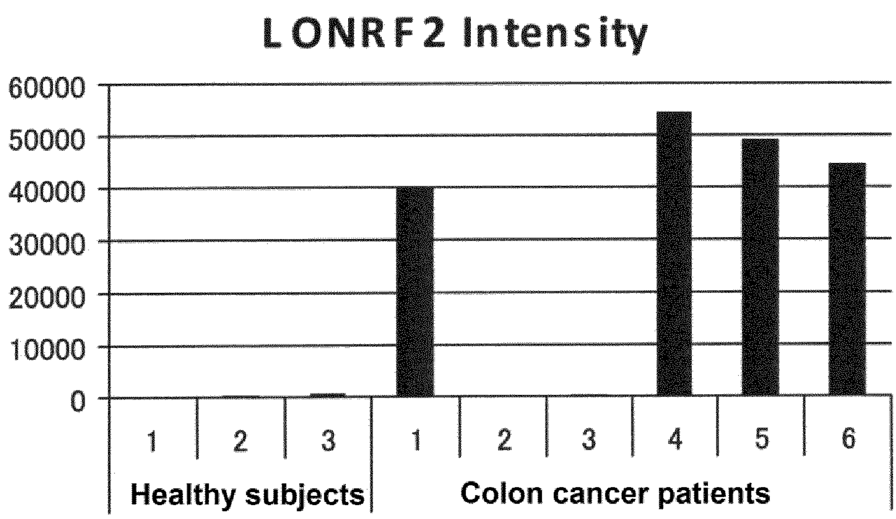

FIG. 6(B)

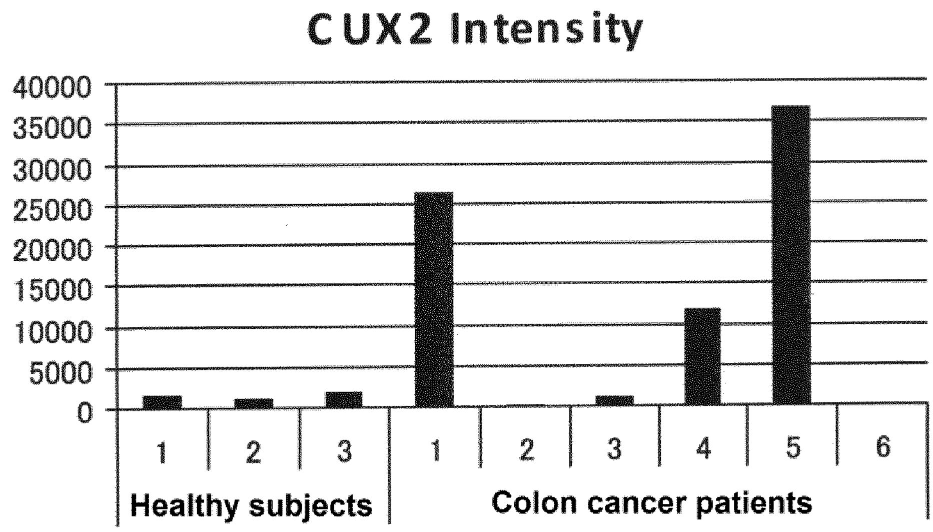
CUX2 Intensity
40000
35000
30000
25000
20000
15000
10000
5000
0
1
2
3
1
2
3
4
5
6
Healthy subjects
Colon cancer patients

FIG. 6(C)

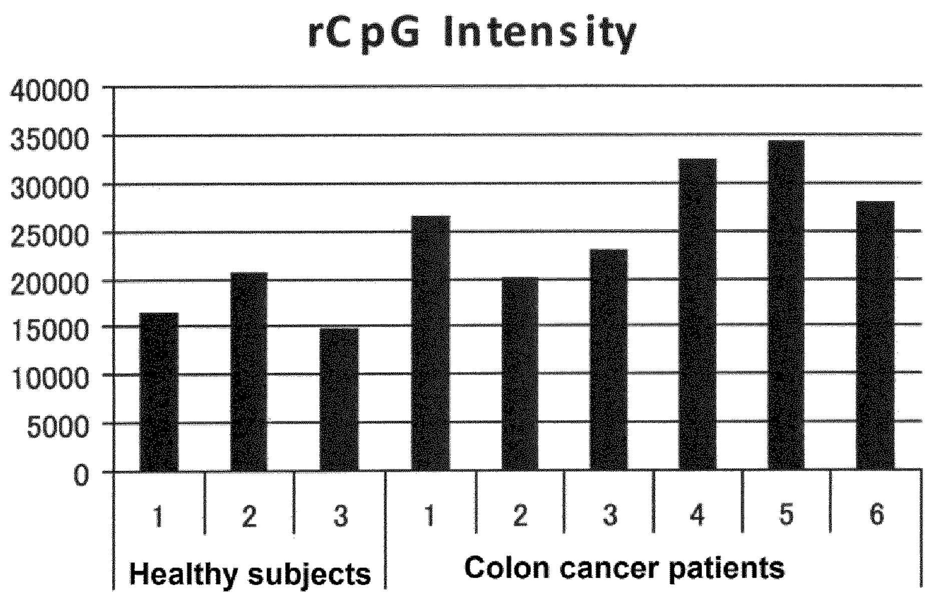
rCpG Intensity
40000
35000
30000
25000
20000
15000
10000
5000
0
1
2
3
1
2
3
4
5
6
Healthy subjects
Colon cancer patients

FIG. 7

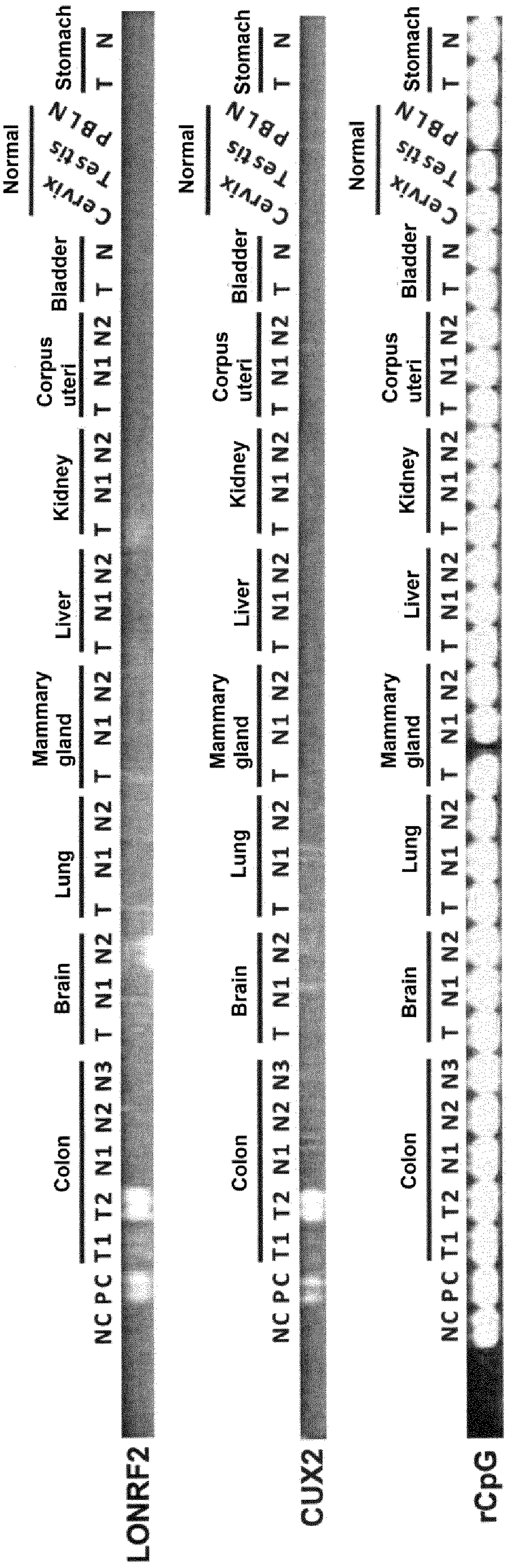
LONRF2
CUX2
rCpG
NC PC T1 T2 N1 N2 N3 T N1 N2 T N1 N2 T N1 N2 T N1 N2 T N1 N2 T N1 N2 T N Cervix Testis PBLN T N
Colon
Brain
Lung
Mammary gland
Liver
Kidney
Corpus uteri
Bladder
Normal
Stomach

FIG. 8(A)

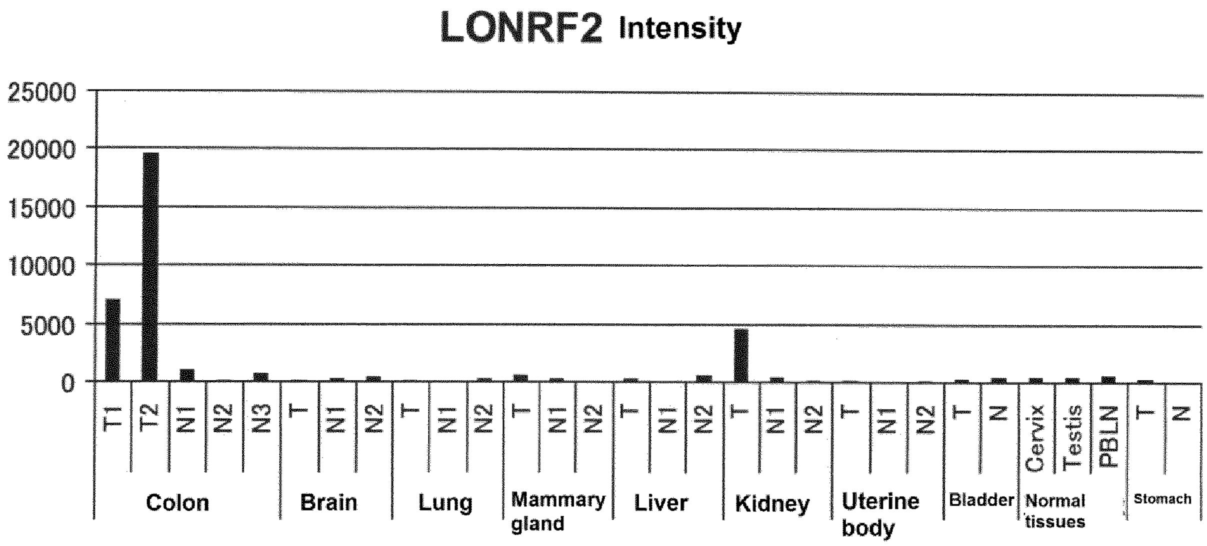
LONRF2 Intensity
25000
20000
15000
10000
5000
0
T1
T2
N1
N2
N3
T
N1
N2
T
N1
N2
T
N1
N2
T
N1
N2
T
N1
N2
T
N1
N2
T
N
Cervix
Testis
PBLN
T
N
Colon
Brain
Lung
Mammary gland
Liver
Kidney
Uterine body
Bladder
Normal tissues
Stomach

FIG. 8(B)

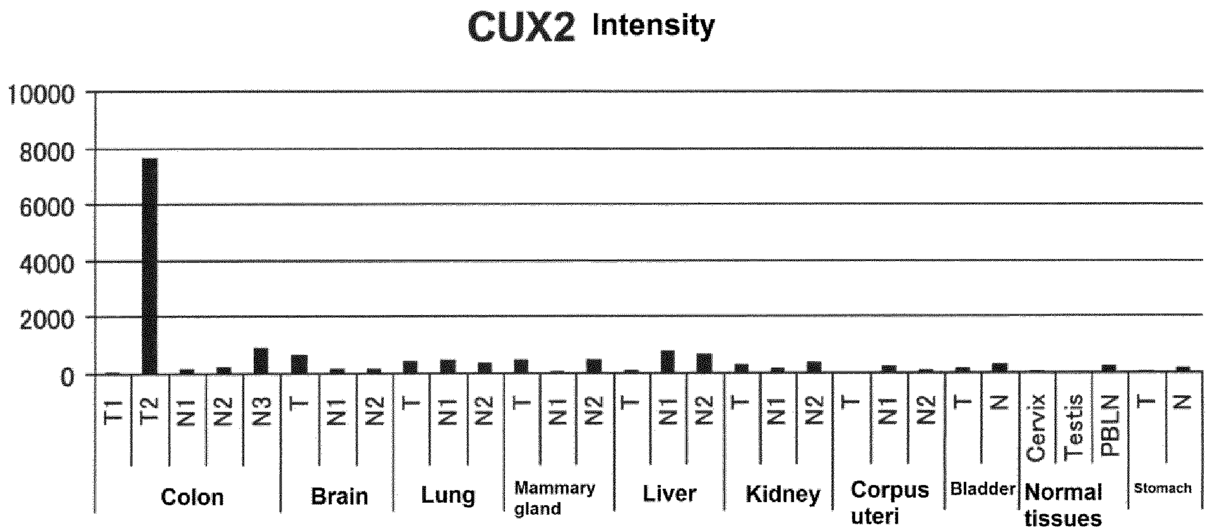
CUX2 Intensity
10000
8000
6000
4000
2000
0
T1
T2
N1
N2
N3
T
N1
N2
T
N1
N2
T
N1
N2
T
N1
N2
T
N1
N2
T
N1
N2
T
N
Cervix
Testis
PBLN
T
N
Colon
Brain
Lung
Mammary gland
Liver
Kidney
Corpus uteri
Bladder
Normal tissues
Stomach

FIG. 8(C)

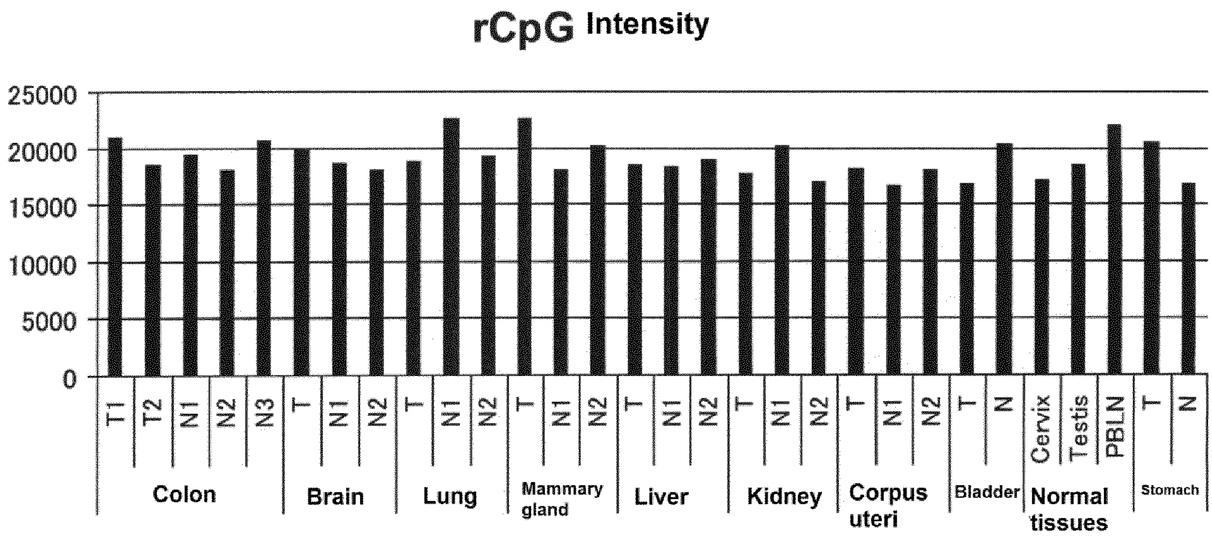
rCpG Intensity
25000
20000
15000
10000
5000
0
T1
T2
N1
N2
N3
T
N1
N2
T
N1
N2
T
N1
N2
T
N1
N2
T
N1
N2
T
N1
N2
T
N
Cervix
Testis
PBLN
T
N
Colon
Brain
Lung
Mammary gland
Liver
Kidney
Corpus uteri
Bladder
Normal tissues
Stomach

FIG. 9

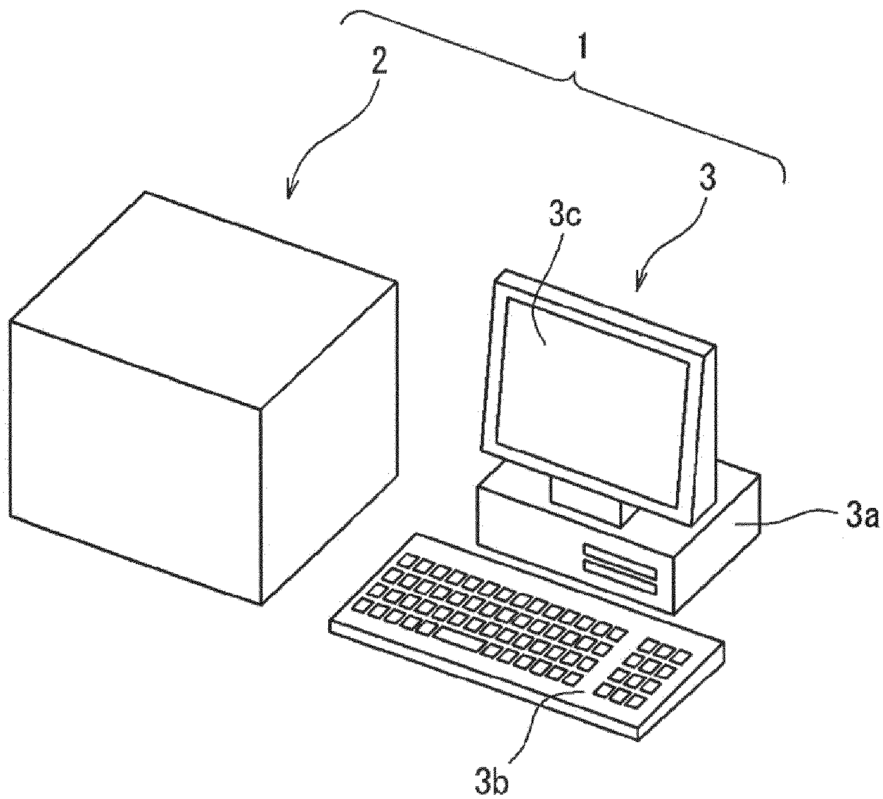
1
2
3
3c
3a
3b

FIG. 10

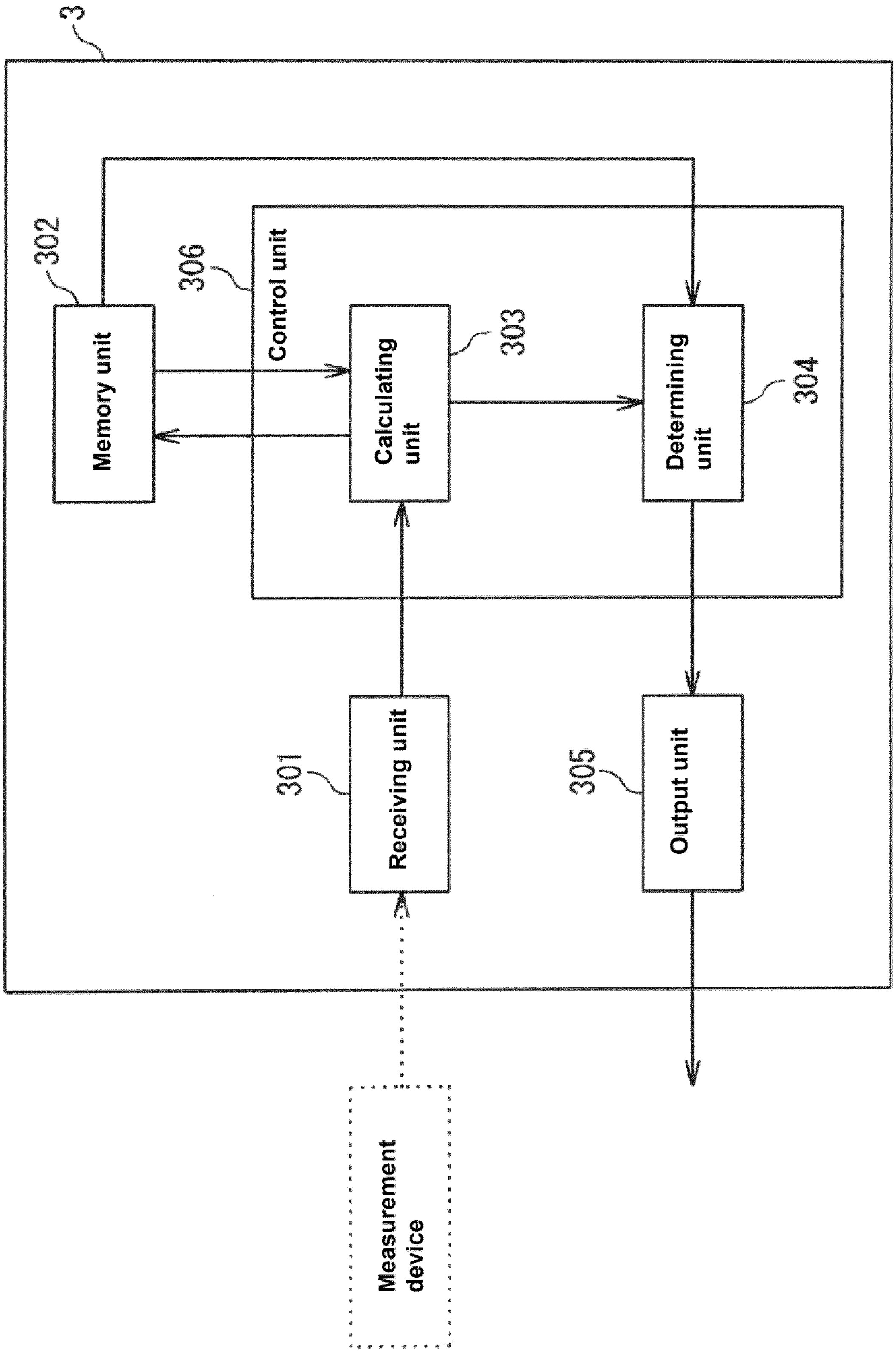
3
302
Memory unit
306
Control unit
Calculating unit
303
Determining unit
304
301
Receiving unit
305
Output unit
Measurement device

FIG. 11

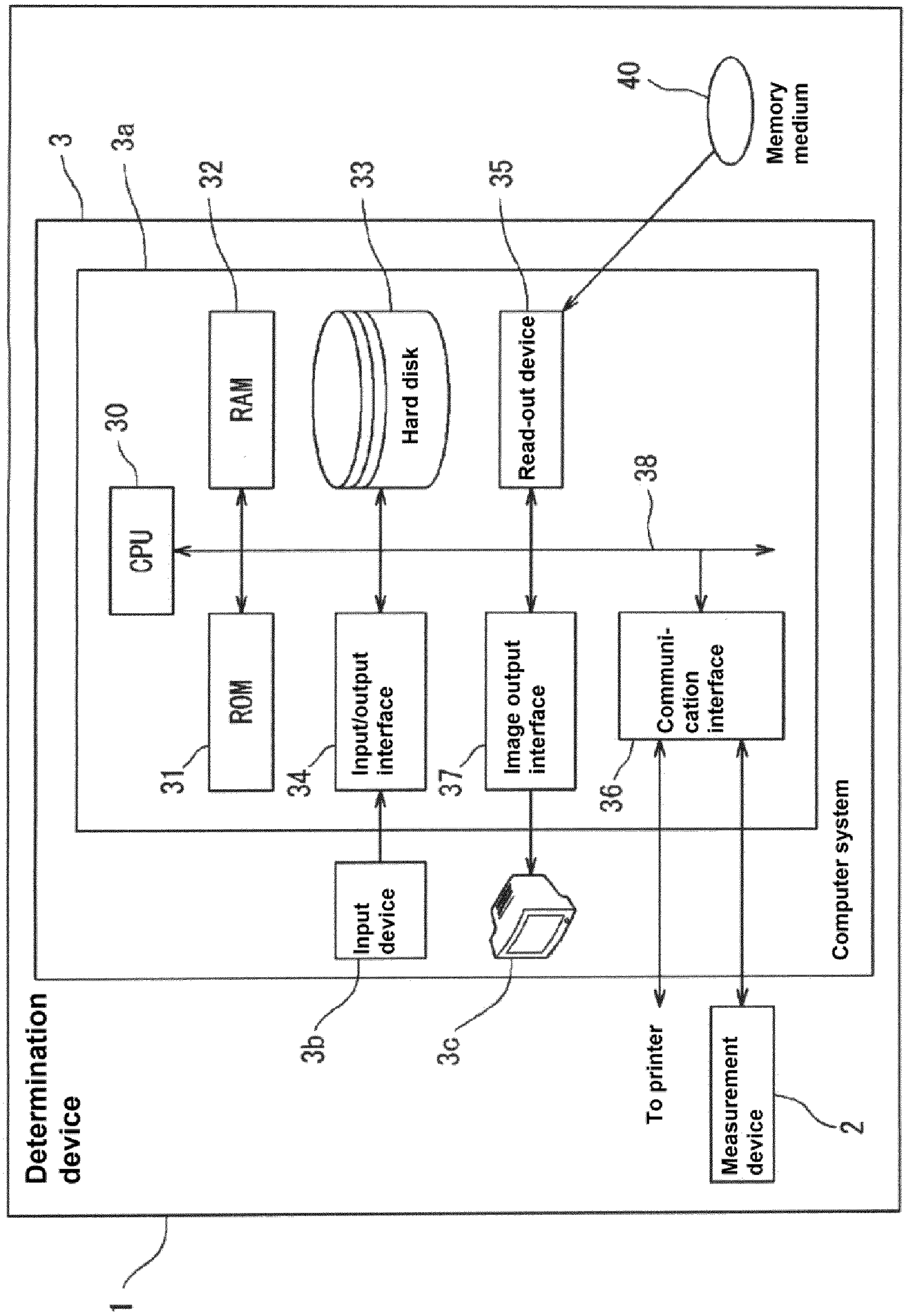
Determination device
1
3
3a
Computer system
CPU
30
ROM
31
RAM
32
Hard disk
33
Input/output interface
34
Read-out device
35
Communi-cation interface
36
Image output interface
37
38
Memory medium
40
Input device
3b
3c
To printer
Measurement device
2

FIG. 12

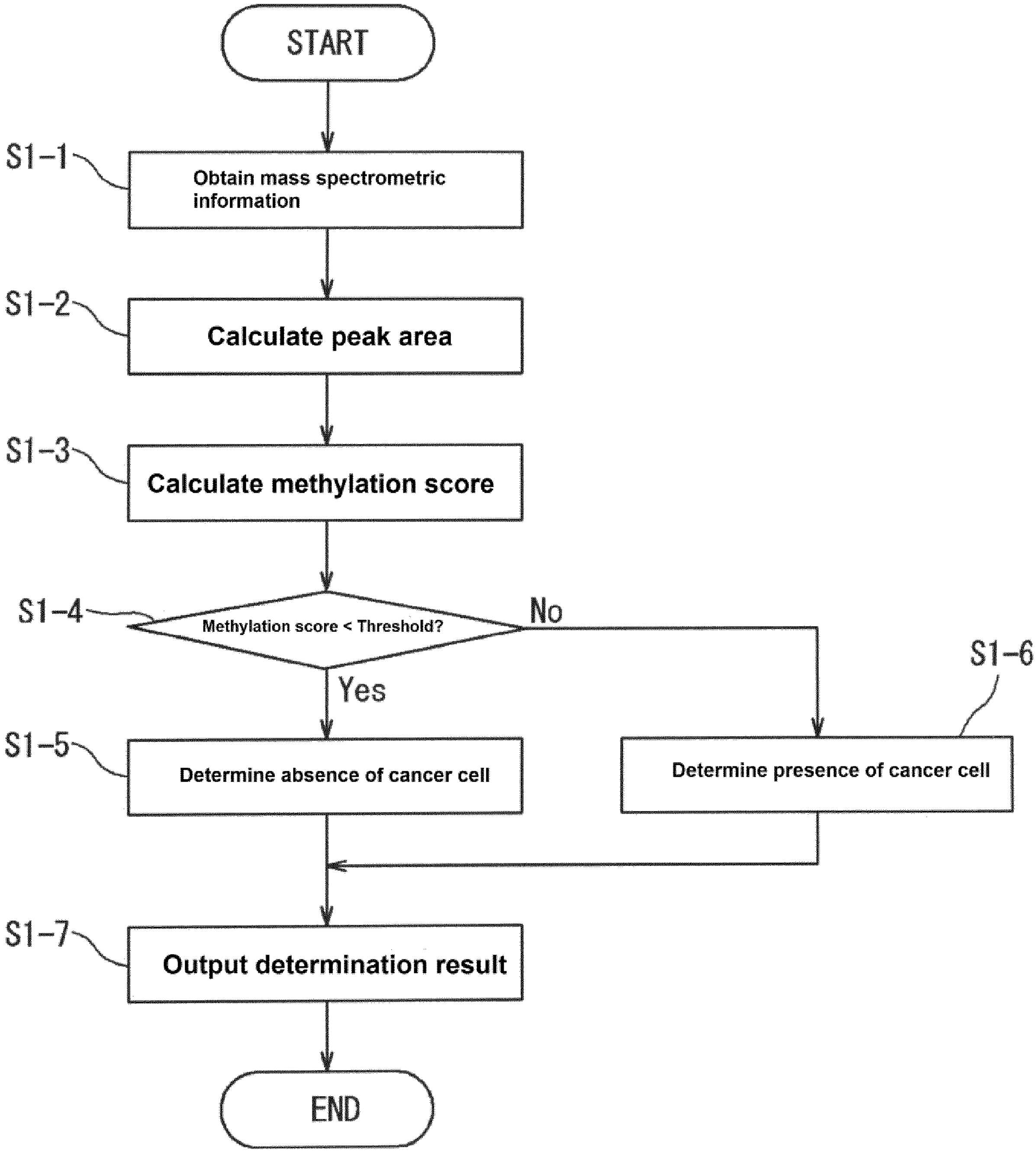
START
S1-1
Obtain mass spectrometric information
S1-2
Calculate peak area
S1-3
Calculate methylation score
S1-4
Methylation score < Threshold?
No
Yes
S1-5
Determine absence of cancer cell
S1-6
Determine presence of cancer cell
S1-7
Output determination result
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013113425 A
- JP 2014093771 A


### Non-patent literature cited in the description


- **KIM et al.** *Annals Surgical Oncol,* 2011, vol. 18 (8), 2338-47
- **KIM et al.** *Cancer Metastasis Rev,* 2010, vol. 29, 181-206
- **HIBI K. et al.** *Jpn. J. Cancer Res.,* 2002, vol. 93, 883-887
- **VILKIN A. et al.** *Cancer. vol.,* 2009, vol. 115, 760-769
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361